# EUROPÄISCHE PATENTSCHRIFT

(11) **EP 1 432 723 B1**
(45) Veröffentlichungstag und Bekanntmachung des Hinweises auf die Patenterteilung: **16.09.2009**
(21) Anmeldenummer: 02800540.3
(22) Anmeldetag: 02.10.2002
(51) Int. Cl.: C07H 21/04

(54) **MODULATION DER EXPRESSION STAT-1-ABHÄNGIGER GENE**
MODULATION OF THE EXPRESSION OF GENES DEPENDENT ON STAT-1
MODULATION DES GENES DEPENDANTS DE L'EXPRESSION STAT-1

(30) Priorität: 04.10.2001 DE 10148828
(43) Veröffentlichungstag der Anmeldung: 30.06.2004
(60) Teilanmeldung: 09009935.9
(73) Patentinhaber: Avontec GmbH, 82152 Martinsried (DE)
(72) Erfinder: HECKER, Markus, 69118 Heidelberg (DE); WAGNER, Andreas, H., 37083 Göttingen (DE)
(74) Vertreter: Dehmel, Albrecht
(86) Internationale Anmeldenummer: PCT/DE2002/003747
(87) Internationale Veröffentlichungsnummer: WO 2003/031459

(56) Entgegenhaltungen:
- WO-A-02/29044
- WO-A-95/08001
- WO-A-95/11687
- KRZESZ R ET AL: "Cytokine-inducible CD40 gene expression in vascular smooth muscle cells is mediated by nuclear factor kappaB and signal transducer and activato of transcription-1" FEBS LETTERS, ELSEVIER SCIENCE PUBLISHERS, AMSTERDAM, NL, Bd. 453, Nr. 1-2, 18. Juni 1999 (1999-06-18), Seiten 191-196, XP004259828 ISSN: 0014-5793
- HUANG JS ET AL: "Role of the Janus kinase (JAK)/signal transducters and activators of transcription (STAT) cascade in advanced glycation end-product-induced cellular mitogenesis in NRK-49F cells." BIOCHEMICAL JOURNAL, Bd. 342, Nr. 1, 15. August 1999 (1999-08-15), Seiten 231-238, XP002242680 ISSN: 0264-6021
- MANN MJ ET AL: "Therapeutic applications of transcription factor decoy oligonucleotides." JOURNAL OF CLINICAL INVESTIGATION, Bd. 106, Nr. 9, November 2000 (2000-11), Seiten 1071-1075, XP002242681 ISSN: 0021-9738
- MORISHITA R ET AL: "Gene therapy in vascular medicine: Recent advances and future perspectives." PHARMACOLOGY & THERAPEUTICS, Bd. 91, Nr. 2, August 2001 (2001-08), Seiten 105-114, XP002242682 August, 2001 ISSN: 0163-7258
- LEE Y J ET AL: "Stat1 alpha expression is involved in IFN-gamma induction of the class II transactivator and class II MHC genes." JOURNAL OF IMMUNOLOGY (BALTIMORE, MD.: 1950) UNITED STATES 15 AUG 1996, Bd. 157, Nr. 4, 15. August 1996 (1996-08-15), Seiten 1559-1568, XP002250394 ISSN: 0022-1767
- STEPHANOU A ET AL: "Ischemia-induced STAT-1 expression and activation play a critical role in cardiomyocyte apoptosis." JOURNAL OF BIOLOGICAL CHEMISTRY, Bd. 275, Nr. 14, 7. April 2000 (2000-04-07), Seiten 10002-10008, XP002250395 ISSN: 0021-9258
- VARGA LIA V ET AL: "Antisense strategies: Functions and applications in immunology" IMMUNOLOGY LETTERS, AMSTERDAM, NL, Bd. 69, Nr. 2, 3. August 1999 (1999-08-03), Seiten 217-224, XP002208684 ISSN: 0165-2478

## Beschreibung

Die vorliegende Erfindung betrifft ein Decoy-Oligonukleotid mit der Nukleinsäuresequenz gemäß SEQ ID NO: 19 oder 20 sowie deren Verwendung als Arzneimittel.

Ein wesentliches Ziel der Dechiffrierung des menschlichen Genoms ist es, krankmachende Gene (aufgrund der Wirkungsweise ihrer Produkte) bzw. krankmachende Veränderungen in der Struktur dieser Gene (Polymorphismen) zu identifizieren und einem Krankheitsbild zuzuordnen. Damit scheint eine Kausaltherapie der meisten Erkrankungen in greifbare Nähe gerückt, wenn man akzeptiert, dass diese durch eine definierte Anzahl von zu stark, zu schwach oder fehlerhaft exprimierten Genprodukten verursacht sind. In der Tat kennt man bereits für eine ganze Reihe von Erbkrankheiten (z.B. Mukoviszidose) den in der Regel singulären Gendefekt (monogenetische Erkrankung), während sich die Situation für andere Erkrankungen (z.B. Bluthochdruck) wesentlich komplexer darstellt. Diese sind offenbar nicht das Resultat eines einzelnen, sondern multipler Gendefekte (polygenetische Erkrankung), welche die betroffenen Personen dazu prädestinieren, beim Zusammentreffen mit bestimmten Umweltfaktoren die Erkrankung zu entwikkeln. Ungeachtet dieser Einschränkung bietet der gezielte Eingriff in die Expression eines oder mehrerer Gene die Chance einer ursachen- und nicht lediglich symptombezogenen Therapie.

Transkriptionsfaktoren sind DNA-bindende Proteine, die sich im Zellkern an die Promotorregion eines oder mehrerer Gene anlagern und dadurch deren Expression, d.h. die Neubildung der Proteine für welche diese Gene kodieren, steuern. Neben der physiologisch wichtigen Kontrolle von Entwicklungs- und Differenzierungsprozessen im menschlichen Körper haben Transkriptionsfaktoren, vor allem wenn sie die Genexpression zum falschen Zeitpunkt aktivieren, ein hohes Krankheitspotenzial. Zusätzlich können (unter Umständen dieselben) Transkriptionsfaktoren Gene mit schützender Funktion blockieren und prädisponierend für die Ausbildung einer Erkrankung wirken. Insofern zielt das im Folgenden beschriebene Anti-Transkriptionsfaktor-Therapieprinzip darauf ab, krankmachende Gene zu hemmen, schützende Gene dagegen einzuschalten.

Die Entzündung ist eine Abwehrreaktion des Organismus und seiner Gewebe gegen schädliche Reize mit dem Ziel, den Schaden zu beheben oder zumindest lokal zu begrenzen sowie die Schadensursache (z.B. eingedrungene Bakterien oder Fremdkörper) zu beseitigen. Auslöser einer Entzündung können Mikroorganismen (Bakterien, Viren, Pilze oder Parasiten), Fremdkörper (Pollen, Asbest- oder Silicatkristalle), Gewebezerstörung durch mechanische Schädigung, chemische Noxen und physikalische Einflüsse sowie durch körpereigene Auslöser (zerfallende Tumorzellen, extravasales Blut, Autoimmunreaktionen) oder Kristalle von im Körper ausgefällten Stoffen (Harnsäure, Calciumoxalat und -phosphat, Cholesterin) sein.

Die rasche Aktivierung von Mastzellen (im Gewebe) oder von basophilen Granulozyten im Blut ist ein Beispiel für die Auslösung einer sehr starken akut-entzündlichen Reaktion und charakteristisch für immunologische Überempfindlichkeitsreaktionen vom Frühtyp (humorale Allergie Typ I). Ist der Organismus bereits vorher mit einem Antigen (bzw. Allergen bei Überempfindlichkeit) in Berührung gekommen, sind als Reaktion darauf B-Lymphozyten sensibilisiert worden, die sich in Kooperation mit zuvor ebenfalls sensibilisierten, CD4-positiven Typ 2 T-Helferzellen (Th2-Zellen) in Plasmazellen umwandeln und Antikörper vom Typ IgE gegen das Antigen bilden. Bei diesem Differenzierungsvorgang ist die Kostimulation der B-Lymphozyten über den CD40-Rezeptor durch die den entsprechenden Liganden (CD154) exprimierenden Th2-Zellen von entscheidender Bedeutung. Binden die Antigen-beladenen IgE-Antikörper an entsprechende Rezeptoren (Typ Fc_{ε}) auf den Mastzellen, so setzen diese verschiedene Entzündungsmediatoren, insbesondere Histamin, Interleukin-8, Leukotriene und Tumornekrosefaktor-α (TNFα) frei. Die Folge ist eine Anlockung professioneller Entzündungszellen, insbesondere von eosinophilen und neutrophilen Granulozyten sowie Monozyten, aber auch von T-Lymphozyten, an den Ort des Geschehens (Chemotaxis). Gleichzeitig kommt es vor allem Histamin-abhängig zu einer Vasodilatation und Permeabilitätssteigerung der die Gefäßwände auskleidenden Endothelzellen. Durch die Gefäßerweiterung sinkt die Strömungsgeschwindigkeit, was den angelockten Leukozyten die physische Kontaktaufnahme mit den Endothelzellen erleichtert. Diese durch Zytokinexposition (z.B. TNFα) bereits aktivierten Endothelzellen exprimieren auf ihrer luminalen Oberfläche verstärkt Selectine (z.B. E-Selectin), die ein Entlangrollen der Leukozyten auf den Endothelzellen und damit die Aktivierung weiterer Adhäsionsmoleküle (Integrine; z.B. *intercellular adhesion molecule-1* [ICAM-1] oder *vascular cell adhesion molecule-1* [VCAM-1]) bewirken. Die Leukozyten können nunmehr an der Gefäßwand haften (Margination) und die Histamin-bedingte Permeabilitätssteigerung (Lockerung des Endothelzellverbandes) begünstigt ihre nachfolgende Auswanderung in den extravasalen Raum (Diapedese). Gleichzeitig gelangt vermehrt proteinreiche Flüssigkeit (entzündliches Exsudat) ins Interstitium, ein Ödem entsteht. Durch den zunehmenden Gewebedruck und durch weitere von den Entzündungszellen gebildete Mediatoren werden umliegende Nervenendigungen gereizt, welche Schmerzen auslösen und damit die Gewebeschädigung bewusst machen.

Die zum Entzündungsort migrierten Granulozyten und die zu Makrophagen umdifferenzierten Monozyten versuchen, die Entzündungsverursacher durch Phagozytose bzw. Lyse zu eliminieren, dabei werden u.a. proteolytische Enzyme und Sauerstoffradikale, die auch das umliegende gesunde Gewebe schädigen können, freigesetzt. Insbesondere die Aktivierung der Makrophagen kann auf vielfältige Weise (z.B. Freisetzung weiterer Zytokine wie Interleukin-1β oder Interleukin-6) dazu beitragen, dass die anfangs lokale Entzündungsreaktion den gesamten Organismus in Form einer Akutphase-Antwort mit einbezieht. Typische Kennzeichen einer Akutphase-Antwort sind Müdigkeit, Abgeschlagenheit und Fieber, eine vermehrte Leukozytenausschüttung aus dem Knochenmark (Leukozytose), der Nachweis von Akutphase-Proteinen im Blut (z.B. C-reaktives Protein), die Stimulation des Immunsystems sowie Gewichtsverlust aufgrund einer veränderten Stoffwechsellage.

Kann die Entzündungsursache beseitigt werden, so schließt sich der Prozess der Wundheilung an, bei dem das zerstörte Gewebe repariert wird. Im günstigsten Fall kommt es zu einer vollständigen Wiederherstellung (Restitutio ad integrum), bei größeren Läsionen oder überschießender Produktion von Bindegewebe (insbesondere Kollagen) resultiert die Bildung einer Narbe, die je nach betroffenen Gewebe unter Umständen mit erheblichen Funktionsstörungen assoziiert ist. Kann die Ursache nicht gleich beseitigt werden (Fremdkörper oder Wundinfektion), verzögert sich die Wundheilung bei gleichzeitiger Intensivierung der Immigration und Aktivität der Phagozyten mit der Folge des Gewebeuntergangs (Nekrose) bis hin zur Höhlenbildung (Abzeß). Das Ergebnis ist fast immer eine narbiger Gewebeumbau mit entsprechendem Funktionsverlust. Gelingt es nicht, diese Erreger-bedingte Entzündung lokal zu begrenzen, breitet sie sich über das Lymphsystem über den gesamten Organismus aus, die Folge ist eine Sepsis mit unter Umständen tödlichen Ausgang (septischer Schock).

Zu einer Störung der Wundheilung kommt es auch dann, wenn sich Entzündungs- und Heilungsprozess die Waage halten. Das Ergebnis ist eine chronische Entzündung, die fibrosierend (übermäßige Kollagensynthese) oder granulomatös (Organisation von Entzündungs-zellen in einem Granulationsgewebe) sein kann, und in der Regel eine kontinuierliche Zerstörung bzw. zunehmende Funktionseinschränkung des betroffenen Gewebes zur Folge hat.

Neben der beschrieben allgemeinen Entzündungsreaktion, die chronisch entarten kann, gibt es Entzündungserkrankungen, die im Hinblick auf die zugrunde liegende Pathogenese Gemeinsamkeiten aber auch distinkte Unterschiede aufweisen. Zwei solcher Entzündungserkrankungen sind beispielsweise Komplikationen nach kardiologisch-chirurgischen Interventionen und die immunologischen Unverträglichkeitsreaktionen, denen aufgrund ihrer enormen klinischen Bedeutung und Variabilität mehr Raum in der Beschreibung gewidmet wird.

Die Ballonkatheter-gestützte mechanische Weitung (perkutane Angioplastie) bzw. die Überbrükkung arteriosklerotisch verengter Arterien mit Hilfe von Venenbypässen stellen bei Patienten mit koronaren bzw. peripheren Durchblutungsstörungen nach wie vor die Therapien der Wahl zum Schutz vor einem drohenden Infarkt oder Organversagen dar. Allerdings ist die Wiederverschlussrate (Restenose) der mechanisch geweiteten und (in der Mehrzahl der Fälle) mit einer metallenen Gefäßstütze (Stent) versorgten Arterien mit 20-50% innerhalb von 6 Monaten inakzeptabel hoch. Auch die Verschlussrate aortokoronarer bzw. peripherer Venenbypässe mit 50-70% nach 5 Jahren ist insbesondere vor dem Hintergrund des periprozeduralen bzw. postoperativen Risikos für die behandelten Patienten mehr als unbefriedigend. Vermutlich durch die mechanische Schädigung der Gefäßwand (hierbei sind Endothelzellen und glatte Gefäßmuskelzellen gleichermaßen betroffen) zeigt die Restenose nach Angioplastie insbesondere im Frühstadium eine ausgeprägte Entzündungskomponente, die u.a. durch die Infiltration professioneller Entzündungszellen (v.a. Monozyten und T-Lymphozyten) in die Gefäßwand gekennzeichnet ist. Auch der fibroproliferativen Stenosierung (Intimahyperplasie) von aortokoronaren bzw. peripheren Venenbypässen scheint eine Entzündungsreaktion zugrunde zu liegen, die insbesondere durch mechanische und physikalische Noxen verursacht ist. Lange bekannt ist auch, dass es beim sogenannten Ischämie/Reperfusionsschaden im Rahmen von chirurgischen Eingriffen oder Organ-transplantationen zu einer entzündungsbedingten Gewebeschädigung kommt, bei der insbesondere die Wechselwirkung von Endothelzellen mit professionellen Entzündungszellen (v.a. Granulozyten, aber auch Monozyten und T-Zellen) sowie die Freisetzung gewebe-schädigender Stoffe (Sauerstoffradikale, Zytokine) eine ganz entscheidende Rolle spielt.

Wichtig im Zusammenhang mit den genannten kardiovaskulären Komplikationen ist, dass es Schutzmechanismen, v.a. in den Endothel- und glatten Muskelzellen der Gefäßwand gibt, die helfen, das Ausmaß der Entzündungsreaktion und des nachfolgenden adaptiven Gewebeumbaus zu begrenzen. Hierzu gehört beispielsweise die Synthese von Stickstoffmonoxid (NO) durch die NO-Synthase in den Endothelzellen. NO, wahrscheinlich in seiner Eigenschaft als endogener Antagonist des Sauerstoffradikals Superoxid, hemmt u.a. die Expression pro-inflammatorischer Chemokine (z.B. *monocyte chemoattractant protein-1,* MCP-1) und Adhäsionsmoleküle (z.B. ICAM-1) in Endothelzellen, die Expression von Rezeptoren für Wachstumsfaktoren in glatten Muskelzellen (z.B. Endothelin B-Rezeptor) sowie die Freisetzung von Wachstumsfaktoren aus Leukozyten. Insofern ist leicht nachzuvollziehen, dass eine mechanische ebenso wie eine funktionelle Schädigung des Endothels (z.B. durch eine Zytokin-induzierte Verminderung der Expression der NO-Synthase in diesen Zellen), die den genannten kardiovaskulären Komplikationen zugrunde liegenden Prozesse von Entzündung und nachfolgendem fibroproliferativen Gefäßwandumbau entgegenwirken.

Alle bisherigen Versuche, die Restenose nach Angioplastie medikamentös einzudämmen, haben bei der Mehrzahl der Patienten nicht die gewünschte Wirkung erzielt. Zur Zeit werden zwei lokale Therapieprinzipien favorisiert: Die bereits zugelassene vaskuläre Brachytherapie, ein Verfahren zur Eindämmung des Zellwachstums durch kurzzeitige radioaktive Bestrahlung des gedehnten Gefäßabschnitts, und die noch in der klinischen Prüfung befindlichen *drug-eluting stents.* Bei diesem Verfahren werden Polymer-beschichtete Stents eingesetzt, welche mit wachstumshemmenden Medikamenten (Zytostatika, Immunsuppressiva) "imprägniert" sind und diese langsam über einen Zeitraum von mehreren Wochen freisetzen. Neueste klinische Studien belegen, dass beide Therapieansätze trotz anfänglich ermutigender Ergebnisse, nicht frei von zum Teil gravierenden Problemen sind (z.B. In-Stentthrombose mit der Gefahr eines Infarktes).

Neben der bereits geschilderten immunologischen Unverträglichkeitsreaktion vom Typ I gibt es prinzipiell vier weitere Allergieformen bzw. Immunregulationsstörungen. Die Typ 1-Reaktion selbst kann nach erfolgter Allergisierung prinzipiell in zwei Phasen eingeteilt werden: die rasche Freisetzung und Neubildung gefäßaktiver Entzündungsmediatoren aus IgE-besetzten Mastzellen und die von den angelockten eosinophilen und neutrophilen Granulozyten vermittelte Spätreaktion. Die Typ 1-Reaktion insgesamt kann in Abhängigkeit von der Allergenexposition lokal oder generalisiert verlaufen. Allergene in der Atemluft lösen Reaktionen im Respirationstrakt, typischerweise mit Schleimhautödem und Hypersekretion (allergische Rhinopathie, Heuschnupfen) sowie Bronchospasmus (Asthma) aus, Nahrungsallergene dagegen in erster Linie Magen-Darm-Symptome wie Übelkeit, Erbrechen und Durchfall. Die Haut reagiert auf Allergene mit Jucken, Schwellung und Nesselsucht (Urtikaria) sowie atopischer Dermatitis (Neurodermitis). Gelangt das Allergen dagegen direkt in die Blutbahn (z.B. Infusion von Blutprodukten, Medikamente) oder ist die Allergenexposition besonders stark, resultiert eine systemische Sofortreaktion, die unter Umständen einen lebensbedrohlichen Blutdruckabfall nach sich zieht (anaphylaktischer Schock).

Bei der Typ II-Reaktion stehen antigen wirksame Zellen (z.B. körperfremde Blutzellen) oder extrazelluläre Proteine (z.B. Medikamenten-induzierte Veränderung der Oberfläche einer körpereigenen Zelle) im Mittelpunkt. Nach der Allergisierung werden beim Zweitkontakt allergenspezifische Antikörper vom Typ IgG und IgM gebildet, die sich in großer Zahl an die Oberfläche der allergenen Zelle binden (Opsonierung). Dadurch wird das Komplementsystem (Bildung eines Membranangriffkomplexes) und eine spezielle Lymphozyten-Subpopulation, die *natural killer cells* (NK-Zellen), aktiviert. Das Ergebnis ist eine Zerstörung der allergene Zelle durch Zytolyse. Eine ähnliche Reaktion wird ausgelöst, wenn sich Autoantikörper an körpereigene Strukturen wie beispielsweise die Basalmembran der Glomeruluskapillaren anheften und dadurch eine rasch progrediente Glomerulonephritis mit drohender Niereninsuffizienz auslösen. Neben den Typ 1 T-Helferzellen (Thl-Zellen, s.u.) sind aktivierte NK-Zellen die Hauptproduzenten von Interferon-γ, einem die Entzündungsreaktion insbesondere durch die Aktivierung von Makrophagen, massiv verstärkenden Zytokins.

Die Typ-III-Reaktion ist durch die Bildung und Ablagerung von Immunkomplexen (Antigen-Antikörper-Komplexe) mit nachfolgender Aktivierung des Komplementsystems und von Phagozyten (Granulozyten, Makrophagen) gekennzeichnet. Sie zirkulieren im Blut und lagern sich sukzessive vor allem in den Kapillaren der Nierenglomeruli, aber auch in Gelenken oder in der Haut ab. Die dadurch ausgelöste Entzündungsreaktion kann u.a. eine (Immunkomplex-) Glomerulonephritis, Gelenkschmerzen sowie Urtikaria zur Folge haben. Auch Infektionen können eine systemische Typ-III-Reaktion auslösen, wenn es dem Immunsystem nicht gelingt, die Erreger (z.B. Streptokokken) vollständig zu eliminieren. Typische lokale Typ-III-Reaktionen sind die sogenannte Arthus-Reaktion nach Impfung in der Haut oder die exogene allergische Alveolitis bei der Ablagerung von Antigen-Antikörper-Komplexen in der Lunge (z.B. Taubenzüchterlunge). Auch der systemische Lupus erythematodes ist eine Typ-III-Reaktion, allerdings im Sinne einer Autoimmunerkrankung durch die Bildung von Autoantikörpern.

Die Typ-rV-Reaktion ist im Gegensatz zu den zuvor genannten Überempfindlichkeitsreaktionen nicht humoral sondern zellgebunden und erreicht ihr Maximum in der Regel erst nach mehreren Tagen (verzögerter Reaktionstyp oder *delayed type hypersensitivity*). Auslöser sind v.a. Proteine eingedrungener Fremdorganismen (Bakterien, Viren, Pilze und Parasiten), andere Fremdproteine (z.B. Gliadin aus dem Weizen bei Zöliakie) sowie Haptene (Medikamente, Metalle [z.B. Nickel bei Kontaktdermatitis], Kosmetika und Pflanzenbestandteile). Auch die primäre Abstoßung transplantierter Organe ist eine Typ-IV-Reaktion. Das Antigen wird von (Gewebe)Makrophagen phagozytiert, prozessiert und naiven T-Helferzellen (CD4-positiv) präsentiert; die Sensibilisierung der T-Helferzellen dauert mehrere Tage. Beim Zweitkontakt wandeln sich die so sensibilisierten T-Helferzellen in Thl-Zellen um; dabei spielt die CD154-vermittelte Kostimulation der antigenpräsentierenden Zelle (diese exprimiert den CD40-Rezeptor) eine wichtige Rolle, da es über diesen Signalweg zur Freisetzung von Interleukin-12 aus den Makrophagen kommt. Interleukin-12 leitet die Differenzierung und Proliferation der T-Helferzellen ein. Die Thl-Zellen ihrerseits regen über bestimmte Wachstumsfaktoren (z.B. GM-CSF) die Monozytenbildung im Knochenmark an, rekrutieren diese mit Hilfe bestimmter Chemokine (z.B. MIF) und aktivieren sie über die Freisetzung von IFNγ. Die daraus resultierende sehr starke Entzündungsreaktion kann körpereigenes (z.B. Tuberkulose) bzw. transplantiertes Gewebe in großem Umfang zerstören. Bei der Transplantatabstoßung sind darüber hinaus CD8-positive zytotoxische T-Zellen beteiligt (Zytolyse), die wie die CD4-postiven Th1-Zellen erst durch vorherige Antigenpräsentation in der Lage sind, ihr Ziel (die fremde Zelloberfläche) zu erkennen und sich entsprechend zu "bewaffnen".

Eine der Typ-IV-Reaktion ähnliche Immunregulationsstörung liegt z.B. der rheumatoiden Arthritis oder der multiplen Sklerose (autoreaktive Th1-Zellen) sowie dem Diabetes mellitus (autoreaktive zytotoxische T-Zellen) zugrunde. Neben einer entsprechenden genetischen Prädisposition (MHC-Proteine, Th1/Th2-Imbalance) spielen bei diesen Autoimmun-erkrankungen neben bakteriellen Superantigenen (z.B. Tbc-Erreger) möglicherweise auch gegen bestimmte Erregerantigene (z.B. Streptokokken) gerichtete T-Zellen eine Rolle, die mit (körpereigenen) Autoantigenen kreuzreagieren (molekulares Mimikry). Typ-V-Reaktionen werden dagegen u.a. durch aktivierende oder blockierende Autoantikörper von Hormon- (z.B. Thyreotropin beim Morbus Basedow) bzw. Neurotransmitter-Rezeptoren (z.B. Acetylcholin bei der Myasthenia gravis) hervorgerufen.

Mit der Transplantatabstoßung vergleichbar, allerdings im umgekehrten Sinne, ist die *graft versus host disease* (GVHD), die im Zuge von allogenen Knochenmarkstransplantationen (zwischen genetisch nicht identischen Individuen) bei circa 40% der Empfänger auftritt. In der bis zu dreimonatigen Akutphase greifen die mit den Stammzellen übertragenen T-Zellen des Spenders den Wirtsorganismus an, die resultierende unter Umständen schwere Entzündungsreaktion manifestiert sich bevorzugt in der Haut, im Gastrointestinaltrakt und in der Leber.

Für die Behandlung akuter Entzündungserkrankungen werden in Abhängigkeit von der mutmaßlichen Ursache im Regelfall nicht steroidale Antiphlogistika (NSAIDs, u.a. Hemmung der Prostaglandin-Synthese) und/oder Antiinfektiva (Abtötung von Bakterien, Pilzen oder Parasiten) bzw. antivirale Chemotherapeutika eingesetzt, eventuell auch Glukokortikoide (generelle Hemmung der Genexpression) lokal. Bei schwerwiegenden oder chronisch rezidivierenden Entzündungserkrankungen werden Glukokortikoide oder Immunsuppressiva (Hemmung der T-Zellaktivierung) oder Zytostatika wie Methotrexat systemisch appliziert. Dies gilt auch für die Transplantation von Organen bzw. Knochenmark. Trotz ihrer unumstrittenen therapeutischen Wirkung kann die systemische Applikation der genannten Arzneimittel insbesondere bei Dauergebrauch schwerwiegende Nebenwirkungen hervorrufen. So entwickeln beispielsweise bis zu 25% der Patienten, die Methotrexat für 2 oder mehr Jahre einnehmen, eine schwere Leberzirrhose. Neuere insbesondere bei chronischen rezidivierenden Entzündungserkrankungen eingesetzte Wirkstoffe blockieren die pro-inflammatorische Wirkung von TNFα: Antikörper gegen das Zytokin selbst bzw. seinen Rezeptor, niedermolekulare Rezeptorantagonisten sowie ein rekombinant hergestelltes, lösliches Rezeptorprotein, welches das Zytokin abfängt. Allerdings mehren sich die Hinweise auf ein verstärktes Auftreten von Infektionserkrankungen unter Therapie mit dem Rezeptorprotein (u.a. Tuberkulose), und circa 40% der Patienten scheinen auf die Therapie gar nicht anzusprechen (*non-responder*). Auch für den zugelassenen humanisierten TNFα-Antikörper gibt es entsprechende Wamhinweise vor dem Auftreten von Infektionen bis hin zur Sepsis 2-4 Jahre nach Therapiebeginn. Beide Wirkstoffe sind darüber hinaus bei einem akuten Entzündungsschub kontraindiziert. Des weiteren sind niedermolekulare Rezeptorantagonisten für Leukotriene zugelassen, die vor allem in der Asthmatherapie Verwendung finden, sowie Hemmstoffe der Cyclooxygenase-2, eine neue Gruppe nicht steroidaler Antiphlogistika (NSAIDs) mit im Vergleich zu den klassischen NSAIDs erheblich reduzierten gastrointestinalen Nebenwirkungen. Darüber hinaus gibt es eine Reihe weiterer, in der Regel humanisierter Antikörper bzw. Antisense-Oligonukleotid-gestützter Ansätze gegen Adhäsionsmoleküle von Leukozyten bzw. Endothelzellen, Zytokin-Rezeptoren von T-Helferzellen oder IgE-Antikörper, die sich in verschiedenen Phasen der klinischen Prüfung befinden. Sieht man einmal von den Glukokortikoiden und den Antiinfektiva als Gruppe ab, so ist den genannten Pharmaka gemeinsam, dass sie sich spezifisch gegen ein therapierelevantes Zielmolekül richten.

Der vorliegenden Erfindung liegt daher die Aufgabe zugrunde, Mittel für eine Prävention oder Therapie überschießender Entzündungsreaktionen und den hiermit assoziierten Folgen für Morbidität und Mortalität der betroffenen Patienten zur Verfügung zu stellen.

Die Aufgabe wird durch den in den Patentansprüchen definierten Gegenstand gelöst.

Die Erfindung wird durch die nachfolgenden Figuren näher erläutert:

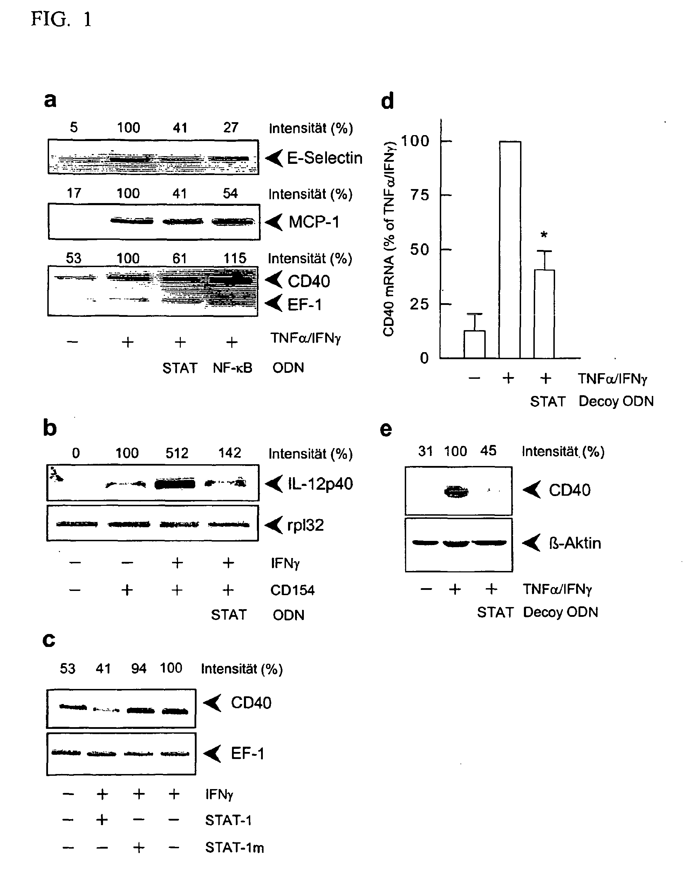

Figur 1 zeigt die Hemmung der Zytokin-stimulierten Expression von CD40 (a, c, d und e), E-Selectin und MCP-1 (a) und der CD40-Ligand-Induktion der interleukin-12p40-Expression (b) in kultivierten humanen Endothelzellen durch Neutralisierung des Transkriptionsfaktors STAT-1 mit Hilfe eines entsprechenden Cis-Element Decoys (SEQ ID NO: 33). (a) Repräsentative RT-PCR-Analyse der E-Selectin, MCP-1 und CD40 mRNA-Expression (zuzüglich der densitometrischen Auswertung ("Intensität"), angegeben in % der stimulierten Kontrolle und bezogen auf den internen Standard EF-1) in Endothelzellen, die für 4 Stunden mit einem STAT-1 (SEQ ID NO: 33) oder NF-κB Cis-Element Decoy (10 µM) vorinkubiert und anschließend für 9 Stunden mit 100 U/ml Tumornekrosefaktor-α und 1000 U/ml Interferon-γ inkubiert worden waren. (b) Repräsentative RT-PCR-Analyse der mRNA-Expression von Interleukin-12p40 (zuzüglich der densitometrischen Auswertung ("Intensität"), angegeben in % der stimulierten Kontrolle und bezogen auf den internen Standard rp132) in Endothelzellen, die für 4 Stunden mit einem STAT-1 Cis-Element Decoy (10 µM; SEQ ID NO: 33) vorinkubiert und anschließend für 12 Stunden mit circa 670000 P3xTB.A7-Zellen/ml (diese Maus-Myelomzellen exprimieren stabil den humanen CD40-Liganden, CD154) und 1000 U/ml Interferon-γ inkubiert worden waren. (c) Repräsentative RT-PCR-Analyse der CD40 mRNA-Expression (zuzüglich der densitometrischen Auswertung ("Intensität"), angegeben in % der stimulierten Kontrolle und bezogen auf den internen Standard EF-1) in Endothelzellen, die für 4 Stunden mit einem STAT-1 Cis-Element Decoy (SEQ ID NO: 33) oder dem entsprechenden Kontroll-Oligonukleotid (STAT-1-25mut) vorinkubiert (Konzentration 10 µM) und anschließend für 9 Stunden mit 100 U/ml Tumornekrosefaktor-α und 1000 U/ml Interferon-γ inkubiert worden waren. (d) Statistische Zusammenfassung von 5 unabhängigen Versuchen zur Wirkung des STAT-1 Cis-Element Decoys (SEQ ID NO: 33) auf die zytokinstimulierte CD40 mRNA-Expression in den kultivierten Endothelzellen (*P<0,05 gegenüber den stimulierten Kontrollzellen). (e) Repräsentative Western Blot-Analyse zuzüglich der densitometrischen Auswertung ("Intensität", angegeben in % der stimulierten Kontrolle und bezogen auf den internen Standard β-Aktin) der Wirkung des STAT-1 Cis-Element Decoys (SEQ ID NO: 33) auf die zytokinstimulierte CD40 Proteinexpression in den kultivierten Endothelzellen nach 24 h. Vergleichbare Ergebnisse wurden in weiteren Versuchen erzielt.

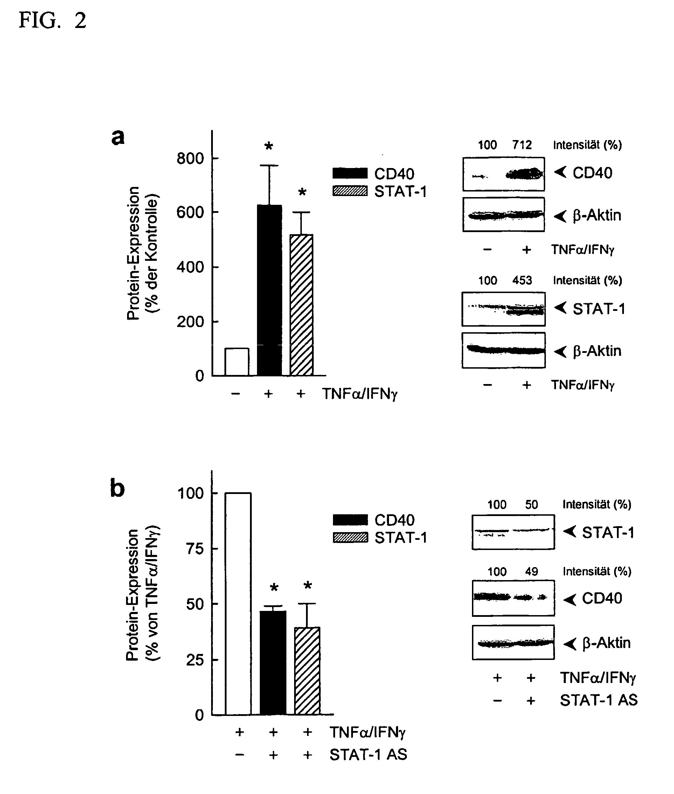

Figur 2 zeigt die Hemmung der Zytokin-induzierten Expression des CD40-Gens in humanen kultivierten Endothelzellen durch die Antisense-Oligonukleotid-gestützte Herunterregulierung der Expression des Transkriptionsfaktors STAT-1. (a) Expression des CD40- bzw. STAT-1-Proteins unter Ruhebedingungen und nach 14stündiger Inkubation der Zellen mit 100 U/ml Tumornekrosefaktor-α und 1000 U/ml Interferon-γ. Die linke Bildhälfte zeigt die statistische Zusammenfassung von 2-4 Versuchen mit unterschiedlichen Zellchargen, die rechte Bildhälfte - zeigt jeweils eine repräsentative Western Blot-Analyse zuzüglich der densitometrischen Auswertung ("Intensität"), angegeben in % der unstimulierten Kontrolle und bezogen auf den internen Standard β-Aktin (*P<0.05 gegenüber den unstimulierten Zellen). (b) Vergleichbare Hemmung der CD40- und STAT-1-Proteinexpression in stimulierten Endothelzellen durch 24stündige Vorbehandlung mit einem STAT-1-Antisense-Oligonukleotide (1 µM; SEQ ID NO: 33). Zusammenfassung von 2 Versuchen (linke Bildhälfte; *P<0.05 gegenüber den stimulierten Kontrollzellen) und repräsentative Western Blot-Analyse (rechte Bildhälfte).

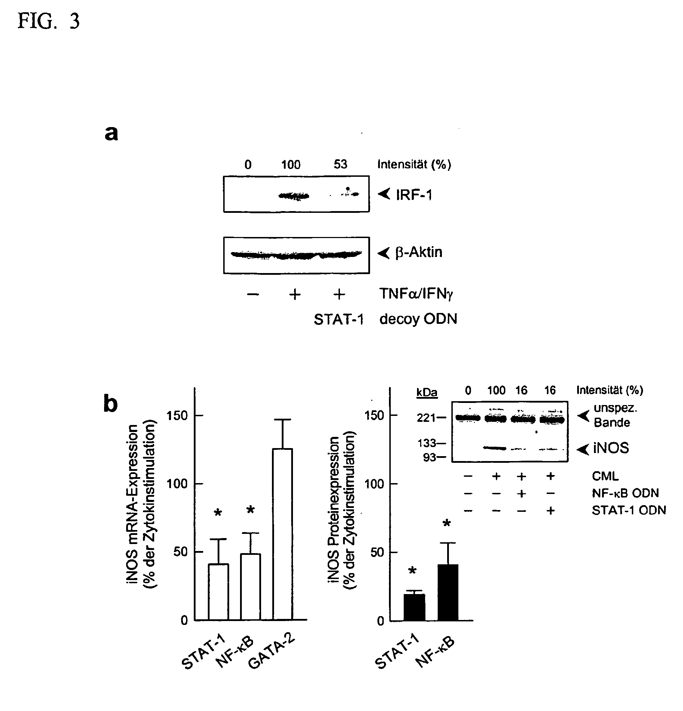

Figur 3 zeigt die Hemmung der Expression des Transkriptionsfaktors IRF-1 in der Monozyten-Zelllinie THP-1 (a) sowie der induzierbaren Isoform der NO-Synthase in kultivierten humanen glatten Muskelzellen (b) durch Neutralisierung des Transkriptionsfaktors STAT-1 mit Hilfe eines entsprechenden Cis-Element Decoys (SEQ ID NO: 33). (a) Repräsentative Western Blot-Analyse zuzüglich der densitometrischen Auswertung ("Intensität"), angegeben in % der stimulierten Kontrolle und bezogen auf den internen Standard β-Aktin. Die kultivierten THP-1-Zellen wurden für 4 Stunden mit dem Cis-Element Decoy (10 µM) vorinkubiert und anschließend für 3 Stunden mit 100 U/ml Tumornekrosefaktor-α und 1000 U/ml Interferon-γ inkubiert. (b) Linke Bildhälfte: Statistische Zusammenfassung von 3 Versuchen mit unterschiedlichen Chargen kultivierter humaner glatter Muskelzellen, die für 4 Stunden mit einem STAT-1 (SEQ ID NO: 33), NF-κB oder GATA-2 Cis-Element Decoy (10 µM) vorinkubiert und anschließend für 9 Stunden mit 1000 U/ml Interferon-γ, 60 U/ml Interleukin-1β, 100 U/ml Tumornekrosefaktor-α und 1 µg/ml bakteriellem Lipopolysaccharid inkubiert worden waren. RT-PCR-Analyse der mRNA-Expression für induzierbare Isoform der NO-Synthase (*P<0.05 gegenüber den stimulierten Zellen = 100%). Rechte Bildhälfte: Statistische Zusammenfassung von 3 Versuchen mit unterschiedlichen Zellchargen und repräsentative Western Blot-Analyse des Hemmung der Zytokin-stimulierten Expression des NO-Synthaseproteins (nach 20 Stunden Exposition) durch Vorinkubation mit dem STAT-1 (SEQ ID NO: 33) bzw. NF-κB Cis-Element Decoy (*P<0.05 gegenüber den stimulierten Zellen = 100%).

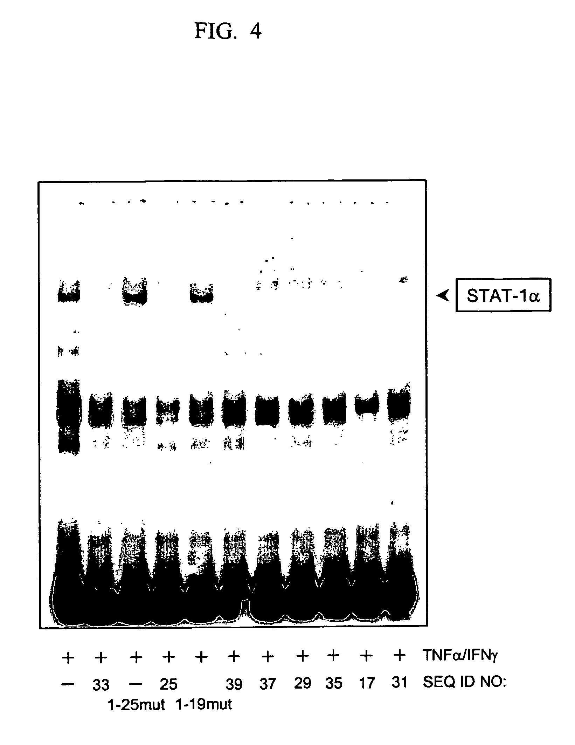

Figur 4 zeigt die Neutralisierung von endogenem STAT-1 in Zellkernextrakten der Monozyten-Zelllinie THP1 durch verschiedene Cis-Element Decoys (SEQ ID NO:17, 25, 29, 31, 33, 35, 37, 39 und die mutierten Kontroll-Oligonukleotide STAT-1-19 mut und STAT-1-25mut). Repräsentative EMSA-Analyse. Kultivierte THP-1 Zellen wurden für 3 Stunden mit 100 U/ml Tumornekrosefaktor-α und 1000 U/ml Interferon-γ inkubiert und im Anschluss für die Herstellung nukleärer Extrakte verwendet. Der Zellkernextrakt wurde zusammen mit dem [³²P]-markierten doppelsträngigen SIE-Oligonukleotid (Santa Cruz Biotechnologie, Heidelberg, Deutschland) und den jeweiligen Cis-Element Decoys bzw. Kontroll-Oligonukleotiden für 20 Minuten bei Raumtemperatur inkubiert und anschliessend der Electrophoretic Mobility Shift-Analyse unterzogen.

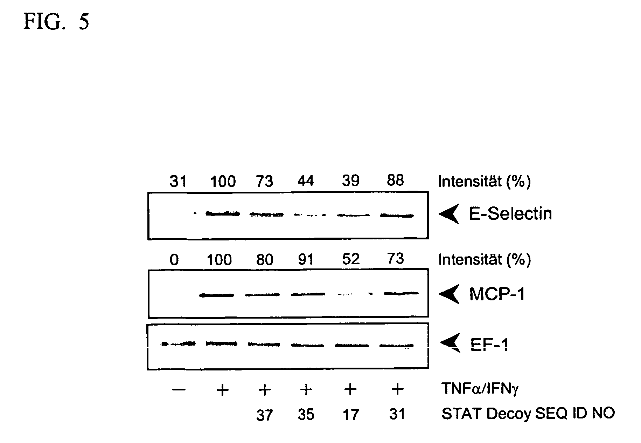

Figur 5 zeigt den Effekt ausgewählter STAT-1 Cis-Element Decoys (SEQ ID NO: 17, 31, 35, 37) auf die Expression von E-Selectin und MCP-1 mRNA in humanen glatten Muskelzellen aus der Thymusvene. Die kultivierten Zellen (Passage 2) wurden für 4 Stunden mit den entsprechenden Cis-Element Decoys (10 µM) vorinkubiert und anschließend für 9 Stunden mit 100 U/ml Tumornekrosefaktor-α und 1000 U/ml Interferon-γ inkubiert. Repräsentative RT-PCR-Analyse, vergleichbare Ergebnisse wurden in weiteren Versuchen erzielt.

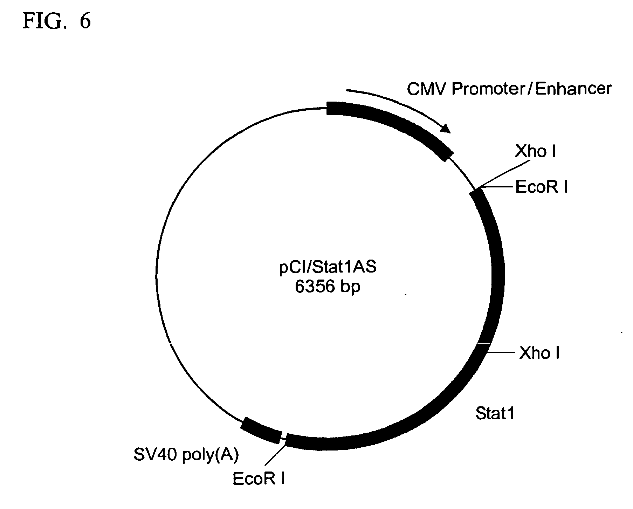

Figur 6 zeigt schematisch die Struktur des STAT-1-Antisense-Expressionsvektors pCI/Statl AS in Form einer Genkarte.

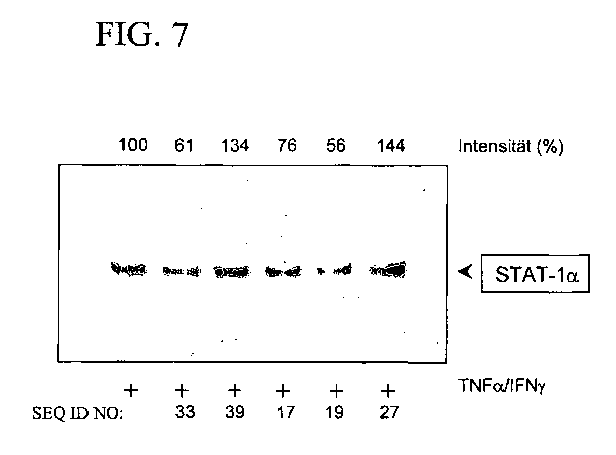

Figur 7 zeigt das Ergebnis der Neutralisierung von STAT-1 in humanen kultivierten Endothelzellen durch verschiedene Cis-Element Decoys (SEQ ID NO: 17, 19, 27, 33 und 39). Repräsentative EMSA-Analyse zuzüglich der densitometrischen Auswertung ("Intensität"). Die kultivierten Endothelzellen wurden für 4 Stunden mit den Decoy-Oligonukleotiden (10 µmol/l) inkubiert und anschließend mit 100 U/ml Tumornekrosefaktor-a und 1000 U/ml Interferon-γ für 30 min stimuliert. Für die EMSA-Analyse wurden die Kernextrakte der stimulierten Zellen und das [32P]-markierte doppelsträngige SIE-Oligonukleotid (Santa Cruz Biotechnologie, Heidelberg, Deutschland) verwendet.

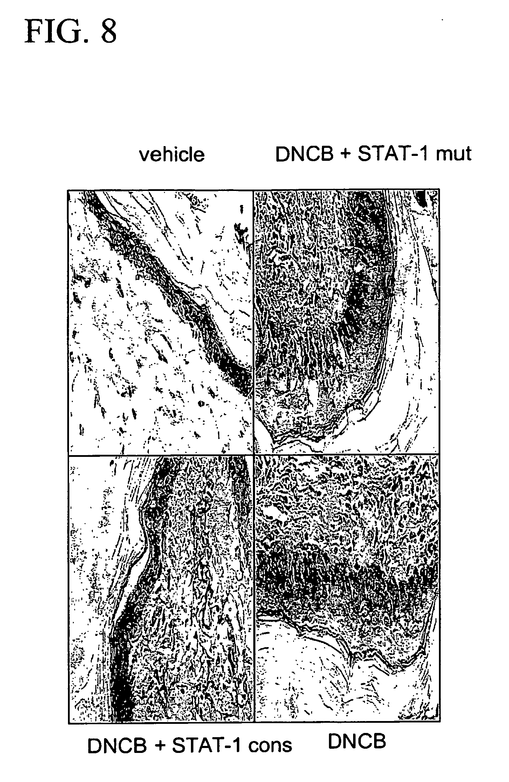

Figur 8 zeigt die histologische Auswertung der Wirkung eines STAT-1 Decoy-Oligonukleotids (STAT-1 cons, 10 nmol, SEQ ID NO: 19) nicht aber eines mutierten Kontroll-Oligonukleotids (STAT-1 mut, 10 nmol, SEQ ID NO: 61) auf die DNCB-induzierte Kontakt-Dermatits in männlichen Meerschweinchen (Original x400, typisches Ergebnis von insgesamt 17 untersuchten Meerschweinchen).

Die Erfinder haben die bei der Zytokin-vermittelten Steigerung der Expression pro-inflammatorischer Genprodukte (CD40, E-Selectin, induzierbare Isoform der NO-Synthase, Interleukin-12 [p40], MCP-1) in humanen Endothel- und glatten Muskelzellen sowie Monozyten beteiligten Transkriptionsfaktoren charakterisiert. Dabei zeigte sich, dass es bei Stimulation der kultivierten Endothelzellen mit TNFα bzw. CD154 in Kombination mit IFNγ zu einem Synergismus zwischen den Transkriptionsfaktoren *nuclear factor κB* (NF-κB) und *signal transducer and activator of transcription-1* (STAT-1) kommt. Ebenso verhielt es sich mit den kultivierten glatten Muskelzellen bzw. Monozyten.

IFNγ alleine war in der Lage, die Expression von CD40 in den humanen Endothelzellen zu steigern, nicht aber die von E-Selectin oder Interleukin-12. Für die Expression dieser beiden von den Endothelzellen kaum bzw. nicht konstitutiv exprimierten Genprodukte ist eine gleichzeitige Stimulation der Zellen mit TNFα (E-Selectin) bzw. CD154 (Interleukin-12) essentiell. Ferner ist für die IFNγ-vermittelte Steigerung der Expression von CD40 nicht aber von E-Selectin in den Endothelzellen und Monozyten die *de novo*-Expression eines weiteren Transkriptionsfaktors, dem *interferon regulatory factor-1* (IRF-1), notwendig. Im Rahmen dieser Untersuchungen zeigte sich, dass die IRF-1-Proteinexpression bei Monostimulation der Zellen mit IFNγ und insbesondere mit TNFα wesentlich schwächer ausfällt als in Gegenwart beider Zytokine. Auch bei der Transkription des IRF-1-Gens wirken demnach die Transkriptionsfaktoren NF-κB und STAT-1 synergistisch zusammen (Ohmori et al., J. Biol. Chem., (1997), 272, 14899).

STAT-1 (GenBank Accession Number NM007315 und XM010893 bzw. http://transfac.gbf.de/cgi-bin/qt/getEntry.pl?t0149) gehört zu einer mindestens 6 Mitglieder umfassenden Gruppe von Transkriptionsfaktoren. Das Produkt des STAT-1-Gens wird von den meisten Zellen konstitutiv exprimiert, liegt aber in der Regel als inaktives monomeres Protein (91 kDa groß) im Zytoplasma vor. Tyrosinphosphorylierung dieser p91-Untereinheit und die nachfolgende Zusammenlagerung (Dimerisierung) zweier solcher p91-Untereinheiten (STAT-1α genannt) ermöglicht den Transport des nunmehr aktiven Transkriptionsfaktors in den Zellkern. Eine Heterodimerisierung mit der p84-Untereinheit von STAT-1β (differentiell gespleißtes Produkt desselben Gens) ist ebenfalls möglich. Die Phosphorylierung der konstitutiv vorhandenen Untereinheiten erfolgt durch zytoplasmatische Januskinasen in Abhängigkeit vom Stimulus. So werden beide Januskinasen (Jak1 und Jak2) durch IFNα stimuliert (besser an den Interferon-Rezeptor rekrutiert); der (patho)physiologisch wichtigste Stimulus für die Aktivierung von STAT-1, IFNγ, stimuliert dagegen nur Jak2. Auch verschiedene Wachstumsfaktoren und Peptidhormone (z.B. Angiotensin II) aktivieren STAT-1; neben den intrinsischen (Wachstumsfaktor)Rezeptortyrosinkinasen spielt hierbei auch eine Mitogen-aktivierte Proteinkinase (MAP-Kinase) eine Rolle. Im Gegensatz zu STAT-1α hat STAT-1β keine transaktivierende, d.h. die Genexpression stimulierende Aktivität.

STAT-1 ist an der Expression einer Reihe potenziell pro-inflammatorischer Genprodukte in Leukozyten, Endothelzellen und glatten Muskelzellen beteiligt, wobei in der Regel die Aktivierung des Transkriptionsfaktors IFNγ-abhängig erfolgt. Ausnahme ist insbesondere die STAT-1-abhängige Expression von Interleukin-6 in Angiotensin II-stimulierten glatten Gefäßmuskelzellen (Schieffer et al., Circ Res, (2000), 87, 1195). Proteine, wozu auch STAT-1 zählt, können auf verschiedenste Weise in ihrer Aktivität inhibiert werden. So können z.B. Anti-STAT-1-Antikörper sowie natürliche oder synthetische Substanzen, welche die STAT-1-Interaktion mit der DNA, d.h. die Transaktivierungsaktivität mindern, verwendet werden. Ferner könnte man die zur STAT-1-Aktivierung führenden Signalwege (Jak1, Jak2, Rezeptortyrosinkinasen, MAP-Kinasen) inhibieren. Bevorzugte Verfahren zur spezifischen Inhibierung der STAT-1-Aktivität sind:
1. Die Neutralisierung (Squelchen) des aktivierten Transkriptionsfaktors durch ein Decoy-Oligonukleotid,
2. die Hemmung der STAT-1-Proteinexpression mit Hilfe eines Antisense-Oligonukleotids, und
3. die Hemmung der STAT-1-Proteinexpression mit Hilfe eines Antisense-Expressionsvektors.

Der hier verwendete Ausdruck "Decoy-Oligonukleotid" oder "Cis-Element Decoy" bezeichnet ein doppelsträngiges DNA-Molekül bzw. ein doppelsträngiges DNA-Oligonukleotid. Die beiden DNA-Stränge weisen eine komplementäre Sequenz auf. In der vorliegenden Erfindung weist das Cis-Element Decoy eine Sequenz auf, die der natürlichen STAT-1 Kernbindungssequenz im Genom entspricht oder ähnelt und an die der Transkriptionsfaktor STAT-1 in der Zelle bindet. Das Cis-Element Decoy wirkt somit als Molekül zur kompetitiven Inhibierung (besser Neutralisierung) von START- 1.

Ein Aspekt der vorliegenden Erfindung besteht in der Bereitstellung eines doppelsträngigen DNA-Oligonukleotids, das in der Lage ist, sequenzspezifisch an den Transkriptionsfaktor STAT-1 zu binden und eine Sequenz gemäß SEQ NO: 19 aufweist,wobei hier nur jeweils ein Strang des doppelsträngigen DNA-Oligonukleotids wiedergegeben ist und der komplementäre Strang ebenfalls umfaßt ist. Nachfolgend sind weitere beispielhafte DNA-Oligonukleotide aufgelistet:
5"NTTNCBGDAAN-3' (SEQ ID NO:1),
5"ATTACCGGAAG-3' (SEQ ID NO:3),
5'-ATTCCGGTAAG-3' (SEQ ID NO:5),
5'-ATTCCTGGAAG-3' (SEQ ID NO:7),
5'-ATTCCTGTAAG-3' (SEQ ID NO:9),
5'-GTTCCAGGAAC-3' (SEQ ID NO:11),
5'-GTTCCCGGAAG-3' (SEQ ID NO:13),
5'-GTTCCGGGAAC-3' (SEQ ID NO:15),
5'-TGTGAATTACCGGAAGTGAGA-3' (SEQ ID NO:17),
5'-TGTGAATTACCGGAAGTG-3' (SEQ ID NO:19),
5'-AGTCAGTTCCAGGAACTGACT-3' (SEQ ID NO:21),
5'-ATGTGAGTTCCCGGAAGTGAACT-3' (SEQ ID NO:23),
5'-ACAGTTCCGGGAACTGTC-3' (SEQ ID NO:25),
5'-GACAGTTCCGGGAACTGTC-3' (SEQ ID NO:27),
5'-GTGTATTCCGGTAAGTGA-3' (SEQ ID NO:29),
5'-TTATGTGAATTCCTGGAAGTG-3' (SEQ ID NO:31),
5'-CATGTTATGCATATTCCTGTAAGTG-3' (SEQ ID NO:33),
5'-TGTGAATTCCTGTAAGTGAGA-3' (SEQ ID NO:35),
5'-TGCATATTCCTGTAAGTG-3' (SEQ ID NO:37),
5'-ATATTCCTGTAAGTG-3' (SEQ ID NO:39).

Wird das erfindungsgemäße Decoy-Oligonukleotid gegen STAT-1, nicht aber ein entsprechendes Kontroll-Oligonukleotid in humanen Endothelzellen verwendet, wird die Zytokin-induzierte Expression von CD40 (sowohl bei Monostimulation mit IFNγ wie auch bei Kombination von IFNγ und TNFα) deutlich um mehr als 50% gehemmt. Dies gilt auch für die Expression von E-Selectin und MCP-1 bzw. Interleukin-12 (p40), wenn die Stimulation der Zellen mit IFNγ und TNFα bzw. CD154 erfolgt. Ein Ausschalten der STAT-1-Aktivität hat demnach eine hochsignifikante Inhibierung der Expression einer Gruppe von pro-inflammatorischen Genprodukten in den humanen Endothelzellen zur Folge. Insofern ist bei diesem Therapieansatz mit einer deutlichen Abschwächung der Endothel-Leukozyten-Wechselwirkung (E-Selectin, MCP-1), aber auch der Wechselwirkung von antigenpräsentierenden Zellen (z.B. Makrophagen und B-Lymphozyten) mit T-Lymphozyten (CD40, Interleukin-12) im Rahmen von Entzündungserkrankungen zu rechnen. Sinngemäß gilt dies auch für die gezeigte Abschwächung der Zytokin-induzierten IRF-1-Expression in den THP-1-Monozyten (und damit der nachgeschalteten Expression IRF-1-abhängiger Gene) sowie der Zytokin-induzierten Expression der genannten Genprodukte einschließlich der induzierbaren NO-Synthase in den humanen glatten Muskelzellen.

Ein bevorzugtes Verfahren zur spezifischen Inhibierung der STAT-1-Aktivität ist daher die Verwendung des erfindungsgemäßen doppelsträngigen DNA-Oligonukleotides, auch Cis-Element Decoy oder Decoy-Oligonukleotid genannt, das eine Bindungsstelle für STAT-1 enthält. Die exogene Zufuhr einer großen Zahl von Transkriptionsfaktor-Bindungsstellen zu einer Zelle, insbesondere in viel höherer Zahl als im Genom vorhanden, erzeugt eine Situation, in der die Mehrzahl eines bestimmten Transkriptionsfaktors spezifisch an das jeweilige Cis-Element Decoy und nicht an seine endogenen Ziel-Bindungsstellen bindet. Dieser Ansatz zur Inhibition der Bindung von Transkriptionsfaktoren an ihre endogene Bindungsstelle wird auch als Squelching bezeichnet. Squelching (oder auch Neutralisation) von Transkriptionsfaktoren unter Verwendung von Cis-Element Decoys wurde erfolgreich eingesetzt, um das Wachstum von Zellen zu inhibieren. Dabei wurden DNA-Fragmente verwendet, die spezifische Transkriptionsfaktor-Bindungsstellen des Transkriptionsfaktors E2F enthielten (Morishita et al., PNAS, (1995) 92, 5855).

Die Sequenz einer Nukleinsäure, die zur Verhinderung der Bindung des Transkriptionsfaktors STAT-1 verwendet wird, ist beispielsweise die Sequenz, an die STAT-1 natürlicherweise in der Zelle bindet. STAT-1 bindet spezifisch an das Motiv mit der Sequenz 5'-NNNSAN TTCCGGGAANTGNSN-3', wobei N = A, T, C oder G und S = C oder G bedeutet. Für eine effektive Bindung von STAT-1 kommt es auf die exakte Übereinstimmung mit den unterstrichenen Basen und den Abstand zwischen diesen Basen an. Daher kann das erfindungsgemäße Cis-Element Decoy folgende 11-mer Konsensus-Kernbindungssequenz aufweisen: 5'-NTTNCBGDAAN-3' (SEQ ID NO:1), wobei B = C, G oder T, D = A, G oder T und N = A, T, C oder G bedeutet. Das Cis-Element Decoy kann ferner größer als die 11-mer Kernbindungssequenz sein und am 5'-Ende und/oder am 3'-Ende verlängert werden. Entsprechende Mutationen im Bereich der Kembindungssequenz führen zum Verlust der Bindung von STAT-1 an das Decoy-Oligonukleotid.

Da das Cis-Element Decoy eine doppelsträngige Nukleinsäure ist, umfaßt das erfindungsgemäße DNA-Oligonukleotid jeweils nicht nur die Sense- oder Forward-Sequenz sondern auch die komplementäre Antisense- oder Reverse-Sequenz. DNA-Oligonukleotide können eine 11-mer-Kernbindungssequenzen für STAT-1 aufweisen, wie sie in SEQ ID NO:3 bis SEQ ID NO: 16 enthalten ist.

Das Cis-Element Decoy kann jedoch auch eine zur vorstehenden Sequenz abweichende Sequenz aufweisen und länger als ein 11-mer sein, z.B. die Sequenzen wie sie in SEQ ID NO:17 bis SEQ ID NO:40 enthalten sind. Diese Cis-Element Decoys enthalten jeweils 2 Bindungsstellen für STAT-1.

Die Bemerkung "2 Bindungsstellen" bezieht sich dabei auf Sense- und Antisense-Strang. Diese Aufzählung der bevorzugten Sequenzen ist nicht abschließend. Dem Fachmann ist ersichtlich, dass eine Vielzahl von Sequenzen als Inhibitor für STAT-1 verwendet werden können, solange sie die vorstehend aufgeführten Bedingungen der 11-mer Konsensus-Kernbindungssequenz und eine Affinität zu STAT-1 aufweisen.

Die Affinität der Bindung einer Nukleinsäuresequenz an STAT-1 kann durch die Verwendung des Electrophoretic Mobility Shift Assay (EMSA) (Sambrook et al. (1989) Molecular Cloning. Cold Spring Harbor Laboratory Press; Krzesz et al. (1999) FEBS Lett. 453, 191) bestimmt werden. Dieses Testsystem ist für die Qualitätskontrolle von Nukleinsäuren, die für die Verwendung in der Methode der gegenwärtigen Erfindung gedacht sind, oder die Bestimmungen der optimalen Länge einer Bindungsstelle geeignet. Sie ist auch für die Identifizierung von anderen Sequenzen, die durch STAT-1 gebunden werden, geeignet. Für einen EMSA, gedacht für die Isolation neuer Bindungsstellen, sind am besten gereinigte oder rekombinant exprimierte Versionen von STAT-1 geeignet, die in mehreren abwechselnden Runden von PCR-Vervielfältigung und Selektion durch EMSA eingesetzt werden (Thiesen und Bach (1990) Nucleic Acids Res. 18, 3203).

Gene, von denen bekannt ist, dass sie STAT-1-Bindungsstellen in ihrem Promotor oder Enhancer-Regionen enthalten oder bei denen es bereits einen funktionellen Nachweis für die Bedeutung von STAT-1 bei ihrer Expression gibt, und die deshalb mutmaßliche Ziele für das spezifische Squelchen durch die Methode der gegenwärtigen Erfindung sind, sind neben dem CD40-, E-Selectin-, induzierbare NO-Synthase, Interleukin-12 (p40)- und MCP-1-Gen weitere pro-inflammatorische Gene, z.B. IFNγ selbst, das Zytokin Interleukin-6, die Adhäsionsmoleküle ICAM-1, PECAM-1 (*platelet endothelial cell adhesion molecule-1*)*,* RANTES (*regulated upon activation, normal T-cell expressed, presumed secreted;* löslich von T-Lymphozyten sezemiert) und VCAM-1 , die Chemokine Interleukin-8, IP-10 (*interferon-inducible protein-10*) und Mig (*monokine induced by gamma-interferon*) sowie die MHC-Proteine I und II. Dabei spielt es keine Rolle, ob die Expression dieser Gene direkt oder indirekt (z.B. durch die STAT-1-abhängige Expression von IRF-1) über STAT-1 reguliert wird.

Die Methode der vorliegenden Erfindung moduliert die Transkription eines Gens oder von Genen in einer solchen Weise, dass das Gen oder die Gene, z.B. E-Selectin, nicht oder vermindert exprimiert werden. Verminderte oder unterdrückte Expression im Rahmen der gegenwärtigen Erfindung bedeutet, dass die Transkriptionsrate verringert ist im Vergleich zu Zellen, die nicht mit einem erfindungsgemäßen doppelsträngigen DNA-Oligonukleotid behandelt werden. Solch eine Verminderung kann beispielsweise durch Northern Blot (Sambrook et al., 1989) oder RT-PCR (Sambrook et al., 1989) bestimmt werden. Typischerweise ist eine solche Verringerung zumindest eine 2-fache, besonders zumindest eine 5-fache, insbesondere zumindest eine 10-fache Verringerung. Der Verlust von Aktivierung kann beispielsweise erreicht werden, wenn STAT-1 an einem bestimmten Gen als Transkriptionsaktivator wirkt und deshalb Squelching des Aktivators zum Verlust der Expression des Zielgens führt.

Darüber hinaus ermöglicht die Methode der vorliegenden Erfindung die Enthemmung der Expression eines Gens, sofern diese von einem konstitutiv aktiven oder (nach entsprechender Stimulation der Zelle) einem aktivierten Transkriptionsfaktor blockiert wird. Ein Beispiel hierfür ist die Enthemmung der Expression des Prepro-Endothelin-1-Gens in nativen Endothelzellen der V. jugularis des Kaninchens durch ein Cis-Element Decoy gegen den Transkriptionsfaktor CCAAT/enhancer binding protein (Lauth et al., J. Mol. Med., (2000), 78, 441). Auf diesem Weg kann die Expression von Genen enthemmt werden, deren Produkte eine schützende Wirkung z.B. gegen Entzündungserkrankungen ausüben. So wird beispielsweise die endotheliale Isoform der NO-Synthase, deren Produkt NO eine entscheidende Rolle bei der Suppression der Expression pro-inflammatorischer Adhäsionsmoleküle und Chemokine in Endothelzellen spielt, durch IFNγ herunterreguliert (Rosenkranz-Weiss et al. (1994) J. Clin. Invest. 93, 1875). Ein Cis-Element Decoy gegen STAT-1 kann diesen unerwünschten Effekt rückgängig machen, indem es die Bindung von STAT-1 an die entsprechende Bindungsstelle im Promoter des endothelialen NO-Synthase-Gens verhindert.

Oligonukleotide werden in der Regel schnell durch Endo- und Exonukleasen, im besonderen DNasen und RNasen in der Zelle, abgebaut. Deshalb können die DNA-Oligonukeotide modifiziert werden, um sie gegen den Abbau zu stabilisieren, so dass über einen längeren Zeitraum eine hohe Konzentration der Oligonukeotide in der Zelle beibehalten wird. Typischerweise kann eine solche Stabilisierung durch die Einführung von einer oder mehrerer modifizierter Internukleotidbindungen erhalten werden.

Ein erfolgreich stabilisiertes DNA-Oligonukleotid enthält nicht notwendigerweise eine Modifikation an jeder Internukleotidbindung. Vorzugsweise sind die Internukleotidbindungen an den jeweiligen Enden beider Oligonukleotide des Cis-Element Decoys modifiziert. Dabei können die letzten sechs, fünf, vier, drei, zwei oder die letzte oder eine oder mehrere Intemukleotidbindungen innerhalb der letzten sechs Internukleotidbindungen modifiziert sein. Ferner können verschiedene Modifikationen der Intemukleotidbindungen in die Nukleinsäure eingeführt werden und die daraus entstehenden doppelsträngigen DNA-Oligonukleotide auf sequenzspezifische Bindung an STAT-1, unter Verwendung des Routine EMSA-Testsystems, getestet werden. Dieses Testsystem erlaubt die Bestimmung der Bindungskonstante des Cis-Element Decoys und so die Bestimmung, ob die Affinität durch die Modifikation verändert wurde. Modifizierte Cis-Element Decoys, die noch eine ausreichende Bindung zeigen, können ausgewählt werden, wobei eine ausreichende Bindung zumindest etwa 50% oder zumindest etwa 75%, und besonders bevorzugt etwa 100% der Bindung der unmodifizierten Nukleinsäure bedeutet.

Cis-Element Decoys mit modifizierter Internukleotidbindung, die immer noch ausreichende Bindung zeigen, können überprüft werden, ob sie stabiler in der Zelle sind als die unmodifizierten Cis-Element Decoys. Die mit Cis-Element Decoys "transfizierten" Zellen werden zu verschiedenen Zeitpunkten auf die Menge der dann noch vorhandenen Cis-Element Decoys untersucht. Dabei wird vorzugsweise ein mit einem Fluoreszenzfarbstoff (z.B. Texas-Rot) markiertes Cis-Element Decoy oder ein radioaktiv markiertes (z.B. ³²P) Cis-Element Decoy eingesetzt mit anschließender digitaler Fluoreszenzmikroskopie bzw. Autoradiographie oder Szintigraphie. Ein erfolgreich modifiziertes Cis-Element Decoy hat eine Halbwertzeit in der Zelle, die höher ist als die eines unmodifizierten Cis-Element Decoys, vorzugsweise von zumindest etwa 48 Stunden, mehr bevorzugt von zumindest etwa 4 Tagen, am meisten bevorzugt von mindestens etwa 7 Tagen.

Geeignete modifizierte Internukleotidbindungen sind in Uhlmann und Peyman ((1990) Chem. Rev. 90, 544) zusammengefaßt. Modifizierte Internukleotid-Phosphat-Reste und/oder Nicht-Phosphor-Brücken in einer Nukleinsäure, die in einer Methode der gegenwärtigen Erfindung eingesetzt werden können, enthalten zum Beispiel Methylphosphonat, Phosphorothioat, Phosphorodithioat, Phosphoramidat, Phosphatester, während Nicht-Phosphor-Internukleotid-Analoge, beispielsweise Siloxan-Brücken, Carbonat-Brücken, Carboxymethylester-Brücken, Acetamidat-Brücken und/oder Thioether-Brücken enthalten. Bei der Verwendung Phosphorothioat-modifizierter Intemukleotidbindungen sollten diese vorzugsweise nicht zwischen den Basen Cytosin und Guanin liegen, da dies zu einer Aktivierung der Zielzellen des Cis-Element Decoys führen kann.

Weiterhin beschrieben ist die Stabilisierung von Nukleinsäuren durch die Einführung struktureller Merkmale in die Nukleinsäure, welche die Halbwertzeit der Nukleinsäure erhöhen. Solche Strukturen, die Haarnadel- und Glocken-DNA enthalten, sind in US 5,683,985 offenbart. Gleichzeitig können modifizierte Internukleotid-Phosphat-Reste und/oder Nicht-Phosphor-Brücken, zusammen mit den genannten Strukturen, eingeführt werden. Die sich daraus ergebenden Nukleinsäuren können im oben beschriebenen Testsystem auf Bindung und Stabilität geprüft werden.

Die Kembindungssequenz kann nicht nur in einem Cis-Element Decoy vorliegen, sondern auch in einem Vektor. Der Vektor kann ein Plasmidvektor sein im besonderen ein Plasmidvektor, der in der Lage ist, autosomal zu replizieren, wodurch er die Stabilität der eingeführten doppelsträngigen Nukleinsäure erhöht.

Das Cis-Element Decoy der vorliegenden Erfindung wird schnell in die Zelle aufgenommen. Eine ausreichende Aufnahme ist durch die Modulation der Expression von einem oder mehreren Genen, die einer Kontrolle durch STAT-1 unterliegen, charakterisiert. Das Cis-Element Decoy der vorliegenden Erfindung moduliert in bevorzugter Weise die Transkription von einem Gen oder Genen nach etwa 4 Stunden der Berührung mit der Zelle, mehr bevorzugt nach etwa 2 Stunden, nach etwa 1 Stunde, nach etwa 30 Minuten und am meisten bevorzugt nach etwa 10 Minuten. Eine typische Mischung, die in so einem Versuch eingesetzt wird, enthält 10 µmol/l Cis-Element Decoy.

Die vorliegende Erfindung betrifft ferner die Verwendung des erfindungsgemäßen Cis-Element Decoys zur Herstellung eines Arzneimittels. Ferner betrifft die vorliegende Erfindung die Verwendung des erfindungsgemäßen Cis-Element Decoys zur Herstellung eines Arzneimittels zur Prävention oder Therapie kardiovaskulärer Komplikationen (z.B. Restenose nach perkutaner Angioplastie, Stenosierung von Venenbypässen), der Transplantatabstoßung, der *graft versus host disease* (GVHD), dem Ischämie/Reperfusionsschaden bei chirurgischen Operationen, von immunologischen Überempfindlichkeitsreaktionen (Typ I bis V), Autoimmunerkrankungen (z.B. Diabetes mellitus, multiple Sklerose, rheumatoide Arthritis) sowie allen anderen Formen akuter, subakuter und chronischer Entzündungserkrankungen, insbesondere der Gelenke (z.B. Arthritis), der Atmungsorgane (z.B. Asthma bronchiale, chronische Bronchitis), der Haut (z.B. Psoriasis, Neurodermitis) und des Gastrointestinaltrakts (z.B. Colitis ulcerosa, Morbus Crohn), einschießlich dem septischen Schock.

Offenbart ist ferner ein Verfahren zur-Modulation der Transkription von mindestens einem Gen in Zellen, insbesondere in Endothelzellen, Epithelzellen, Leukozyten, glatten Muskelzellen, Keratinozyten oder Fibroblasten, wobei die Methode den Schritt der Kontaktierung der genannten Zellen mit einer Mischung, enthaltend eine oder mehrere erfindungsgemäße doppelsträngige Nukleinsäure(n), die in der Lage sind, sequenzspezifisch an den Transkriptionsfaktor STAT-1 zu binden, umfasst. Ein bevorzugtes Verfahren ist z.B. die *ex vivo* Behandlung einer T-Lymphozyten enthaltenen Knochenmarksspende vor Einbringen in den Körper des Empfängers.

Das erfindungsgemäße Cis-Element Decoy kann ferner in einer Zusammensetzung den Patienten verabreicht werden oder in dem offenbarten Verfahren verwendet werden. Die Zusammensetzung (im folgenden Mischung genannt) enthaltend die erfindungsgemäßen Cis-Element Decoys wird mit den Zielzellen (z.B. Endothelzellen, Epithelzellen, Leukozyten, glatten Muskelzellen, Keratinozyten oder Fibroblasten) in Berührung gebracht. Das Ziel dieses In-Berührung-Bringens ist die Übertragung der Cis-Element Decoys, die STAT-1 binden, in die Zielzelle (d.h., die STAT-1-abhängig pro-inflammatorische Genprodukte exprimierende Zelle). Deshalb können Nukleinsäure-Modifikation und/oder Zusatzstoffe oder Hilfsstoffe, von denen bekannt ist, dass sie die Durchdringung von Membran erhöhen, im Rahmen der gegenwärtigen Erfindung benutzt werden (Uhlmann und Peyman (1990) Chem. Rev. 90, 544).

Eine Mischung kann nur Nukleinsäure und Puffer enthalten. Eine geeignete Konzentration des Cis-Element Decoy liegt im Bereich von zumindest 0,1 bis 100 µM, vorzugsweise bei 10 µM, wobei ein oder mehrere geeignete Puffer zugesetzt werden. Ein Beispiel eines solchen Puffers ist Ringer-Lösung enthaltend 145 mmol/l Na⁺, 5 mmol/l K⁺, 156 mmol/l Cl⁻, 2 mmol/l Ca²⁺, 1 mmol/l Mg²⁺, 10 mmol/l Hepes, 10 mmol/l D-Glucose, pH 7,4.

Die Mischung kann zusätzlich mindestens einen Zusatzstoff und/oder Hilfsstoff enthalten. Zusatzstoffe und/oder Hilfsstoffe wie Lipid, kationische Lipide, Polymere, Liposomen, Nanopartikel, Nukleinsäure-Aptamere, Peptide und Proteine, die an DNA gebunden sind, oder synthetische Peptid-DNA-Moleküle sind beabsichtigt, um beispielsweise die Einbringung von Nukleinsäuren in die Zelle zu erhöhen, um die Mischung auf nur eine Untergruppe von Zellen zu richten, um den Abbau der Nukleinsäure in der Zelle zu verhindern, um die Lagerung der Nukleinsäuremischung vor der Verwendung zu erleichtern. Beispiele für Peptide und Proteine oder synthetische Peptid-DNA-Moleküle sind z.B. Antikörper, Antikörperfragmente, Liganden, Adhäsionsmoleküle, die alle modifiziert oder unmodifiziert sein können.

Zusatzstoffe, die Cis-Element Decoys in der Zelle stabilisieren, sind beispielsweise Nukleinsäure-kondensierende Substanzen wie kationische Polymere, Poly-L-Lysin oder Polyethylenimin.

Die Mischung, die in dem beschriebenen Verfahren eingesetzt wird, wird bevorzugt lokal angewendet durch Injektion, Katheter, Suppositiorium ("Zäpfchen"), Aerosole (Nasen- bzw. Mundspray, Inhalation) Trokars, Projektile, pluronische Gele, Polymere, die anhaltend Medikamente freisetzen, oder jede andere Vorrichtung, die lokalen Zugang ermöglicht. Auch die ex vivo Anwendung der Mischung, verwendet im offenbarten Verfahren, erlaubt einen lokalen Zugang.

Die folgenden Figuren und Beispiele dienen nur der Erläuterung und beschränken in keiner Weise den Umfang der Erfindung.

### 1. Zellkultur

Humane Endothelzellen wurden durch Behandlung mit 1,6 U/ml Dispase in Hepes- modifizierter Tyrodelösung für 30 Min. bei 37°C aus Nabelschnurvenen isoliert und auf Gelatine-beschichteten 6-Loch-Gewebekulturschalen (2 mg/ml Gelatine in 0,1 M HCl für 30 Min. bei Umgebungstemperatur) in 1,5 ml M199 Medium (Gibco Life Technologies, Karlsruhe, Deutschland), enthaltend 20% fötales Kälberserum, 50 U/ml Penicillin, 50 µg/ml Streptomycin, 10 U/ml Nystatin, 5 mM HEPES und 5 mM TES, 1 µg/ml Heparin und 40 µg/ml endothelialer Wachstumsfaktor, kultiviert. Sie wurden durch ihre typische Pflasterstein-Morphologie, positive Immunfärbung für von Willebrandt-Faktor (vWF) und fluorimetrischen Nachweis (FACS) von PECAM-1 (CD31) sowie negative Immunfärbung für glattmuskuläres α-Actin (Krzesz et al. (1999) FEBS Lett. 453, 191) identifiziert. Die humane Monozyten-Zelllinie THP-1 (ATCC TIB 202) wurde in RPMI 1640 Medium (Life Technologies), enthaltend 10% fötales Kälberserum, 50 U/ml Penicillin, 50 µg/ml Streptomycin und 10 U/ml Nystatin, kultiviert. Die humanen glatten Muskelzellen wurden aus präparierten Thymusvenen mit Hilfe der Explant-Technik isoliert (Krzesz et al., (1999) FEBS Lett. 453,191) und auf Gelatine-beschichteten 6-Loch-Gewebekulturschalen (s.o.) in 1,5 ml Dulbecco's Modified Eagle Medium, enthaltend 15% fötales Kälberserum, 50 U/ml Penicillin, 50 µg/ml Streptomycin und 10 U/ml Nystatin, kultiviert. Sie wurden durch positive Immunfärbung für glattmuskuläres α-Actin identifiziert.

### 2. RT-PCR-Analyse

Die endotheliale gesamt-RNA wurde mit dem Qiagen RNeasy Kit (Qiagen, Hilden, Deutschland) isoliert, daran anschließend wurde eine cDNA-Synthese mit einem Maximum von 3 µg RNA und 200 U Superscript^{™} II Reversetranskriptase (Life Technologies) in einem Gesamtvolumen von 20 µl entsprechend der Herstelleranleitung durchgeführt. Für den Abgleich der cDNA-Beladung wurden 5 µl (ungefähr 75 ng cDNA) der resultierenden cDNA-Lösung und dem Primerpaar (Gibco) für Elongationsfaktor 1 (EF-1) PCR mit 1 U Taq DNA Polymerase (Gibco) in einem Gesamtvolumen von 50 µl benutzt. EF-1 diente als interner Standard für die PCR. Die PCR-Produkte wurden auf 1,5% Agarose-Gelen enthaltend 0,1% Ethidiumbromid separiert und die Intensität der Banden wurde densitometrisch mit einem CCD-Kamerasystem und der One-Dscan Gelanalyse-Software von Scanalytics (Billerica, MA, USA) bestimmt, um in nachfolgenden PCR-Analysen das Volumen der cDNA anzupassen.

Alle PCR-Reaktionen wurden einzeln für jedes Primer-Paar in einem Hybaid OmnE Thermocycler (AWG; Heidelberg, Deutschland) durchgeführt. Die einzelnen PCR-Bedingungen für die cDNA von humanen Nabelschnurendothelzellen waren wie folgt: CD40 (Produktgröße 381 bp, 25 Zyklen, Anlagerungstemperatur 60°C, (Vorwärtsprimer) 5'-CAGAGTTCACTGAAACGGAATGCC-3' (SEQ ID NO:44), (umgekehrter Primer) 5'-TGCCTGCCTGTTGCACAACC-3' (SEQ ID NO:45)); E-Selectin (Produktgröße 304 bp, 33 Zyklen, Anlagerungstemperatur 60°C, (Vorwärtsprimer) 5'-AGCAAGGCATGATGTTAACC-3' (SEQ IN NO:46), (umgekehrter Primer) 5'-GCATTCCTCTCTTCCAGAGC-3' (SEQ NO:47)); EF-1 (Produktgröße 220 bp, 22 Zyklen, Anlagerungstemperatur 55°C, (Vorwärtsprimer) 5'-TCTTAATCAGTGGTGGAAG-3' (SEQ ID NO:48), (umgekehrter Primer) 5'-TTTGGTCAAGTTGTTTCC-3' (SEQ ID NO:49)); IL-12p40 (Produktgröße 281 bp, 30 Zyklen, Anlagerungstemperatur 62°C, (Vorwärtsprimer) 5'-GTACTCCACATTCCTACTTCTC-3'(SEQ ID NO:50), (umgekehrter Primer) 5'-TTTGGGTCTATTCCGTTGTGTC-3'(SEQ ID NO:51)); rp132 (Produktgrösse 368 bp, 20 Zyklen, Anlagerungstemperatur 60°C, (Vorwärtsprimer) 5'-GTTCATCCGGCACCAGTCAG-3' (SEQ ID NO:52), (umgekehrter Primer) 5'-ACGTGCACATGAGCTGCCTAC-3' (SEQ ID NO:53); MCP-1 (Produktgröße 330 bp, 22 Zyklen, Anlagerungstemperatur 63°C, (Vorwärtsprimer) 5'-GCGGATCCCCTCCAGCATGAAAGTCTCT-3' (SEQ ID NO:54), (umgekehrter Primer) 5'-ACGAATTCTTCTTGGGTTGTGGAGTGAG-3' (SEQ ID NO:55).

### 3. Electrophoretic Mobility Shift Assay (EMSA)

Die nukleären Extrakte und [³²P]-markierten doppelsträngigen Konsensus-Oligonukleotide (Santa Cruz Biotechnologie, Heidelberg, Deutschland), nicht-denaturierende Polyacrylamid-Gelelektrophorese, Autoradiographie und Supershift-Analyse wurden wie bei Krzesz et al. (1999) FEBS Lett. 453, 191 beschrieben durchgeführt. Dabei wurde ein doppelsträngiges DNA-Oligonukleotid mit der folgenden einzelsträngigen Sequenz verwendet (Kernbindungssequenz ist unterstrichen): SIE, 5'-GTGCATTTCCCGTAAATCTTGTC-3' (SEQ ID NO:56). Für die Analyse der Verdrängung von endogenem STAT-1 in Kemextrakten zytokin-stimulierter THP-1-Zellen (prämoozytäre humane Zelllinie) durch die verschiedenen Cis-Element Decoys wurde im EMSA-Bindungsansatz ein Verhältnis von 30:1 (STAT-1 Cis-Element Decoy:[³²-P]-markiertes SIE Oligonukleotid (11 fmol)) gewählt.

### 4. Decoy-Oligonukleotid-Technik

Doppelsträngige Decoy-Oligonukleotide wurden von den komplementären einzelsträngigen Phosphorothioat-verbundenen Oligonukleotiden (Eurogentec, Köln, Deutschland) wie bei Krzesz et al. (1999) FEBS Lett. 453, 191 beschrieben hergestellt. Die kultivierten humanen Endothelzellen wurden für 4 Stunden bei einer Konzentration von 10 µM des jeweiligen Decoy-Oligonukleotids vorinkubiert. Dies waren die Bedingungen, die bereits vorher aufgrund von EMSA und RT-PCR-Analyse optimiert wurden. Danach wurde das Decoy-Oligonukleotid-haltige Medium in der Regel durch frisches Medium ersetzt. Die einzelsträngigen Sequenzen der Oligonukleotide waren wie folgt (unterstrichene Buchstaben kennzeichnen Phosphorothioat-verbundene Basen, alle in 5' - 3' Richtung):

| | |
|---|---|
| GATA-2, | CACTTGATAACAGAAAGTGATAACTCT (SEQ ID NO:57) |
| NF-κB, | AGTTGAGGGGACTTTCCCAGGC (SEQ ID NO:58); |
| STAT-1, | CATGTTATGCATATTCCTGTAAGTG (SEQ ID NO:33); |
| STAT-1-19mut, | GACAGTGCAGTGAACTGTC (SEQ ID NO:59); |
| STAT-1-25mut, | CATGTTATGCAGACCGTAGTAAGTG (SEQ ID NO:60). |

### 5. Antisense-Oligonukleotid (ODN)-Technik

Für einen Antisense-Ansatz wurden 100 µl ml OPTI-MEM^{®}I Kulturmedium mit 15 µl Lipofectin (Gibco Life Technologie, Karlsruhe, Deutschland) versetzt und für 45 Minuten bei Raumtemperatur (RT) inkubiert (Lösung A). Im Anschluss daran wurde das Antisense-ODN (Eurogentec, Köln, Deutschland) in einer finalen Konzentration von 0,5 µM in 100 µl OPTI-MEM^{®}I Kulturmedium hinzugegeben (Lösung B). Nach dem Vereinigen der Lösungen A und B folgte eine weitere Inkubation von 15 Minuten (RT). Bei Versuchsbeginn wurden 0.8 ml des herkömmlichen Zellkulturmediums der Endothelzellkultur (ohne Heparin und endothelialer Wachstumsfaktor) in das Eppendorf-Gefäss, das die Lipofectin-Antisense-ODN-Komplexe enthielt, hinzugegeben und das Zellkulturmedium der Endothelzellkultur durch das Antisense-Lipofectin-Medium ersetzt. Nach 4 Stunden wurde das Antisense-Lipofectin-Medium entfernt und durch frisches Zellkulturmedium (mit Heparin und endothelialem Wachstumsfaktor) ersetzt. Die Sequenz des STAT-1-Antisense-ODN war 5'-T*A*CCA*C*T*G*A*G-*A*C*A*T*CC*T*G-CC*A*C-3' (* Phosphorothioate-modifizierte Base ; SEQ ID NO:41).

### 6. Western Blot-Analyse

Die humanen Nabelschnurendothelzellen und glatten Muskelzellen aus der Thymusvene wurden durch fünfmaliges aufeinanderfolgendes Einfrieren in flüssigem Stickstoff und Auftauen bei 37°C (Heizblock, Kleinfelden) aufgeschlossen. Protein-Extrakte wurden wie bei Hecker et al. (1994) Biochem J. 299, 247 beschrieben hergestellt. 20-30 µg Protein wurden mit Hilfe einer 10%igen Polyacrylamid-Gelelektrophorese unter denaturierenden Bedingungen in der Gegenwart von SDS nach Standardprotokoll aufgetrennt und auf eine BioTrace™ Polyvinylidene Fluoride Transfermembran (Pall Corporation, Roßdorf, Deutschland) transferiert. Zum immulologischen Proteinnachweis wurden folgende primäre Antikörper verwendet: CD40 Protein (polyklonal, 1:2000 Verdünnung, Research Diagnostics Inc., Flanders NJ, USA), STAT-1 Protein (monoklonal, 1:5000 Verdünnung, BD Transduction Laboratories, Heidelberg, Deutschland), IRF-1 Protein (polyklonal, 1:2000 Verdünnung, Santa Cruz Biotechnology, Heidelberg, Deutschland), iNOS Protein (polyklonal, 1:3000 Verdünnung, BD Transduction Laboratories, Heidelberg, Deutschland). Die Proteinbanden wurden nach Hinzufügen eines Peroxidase-gekoppelten-Anti-Kaninchen-IgG bzw. bei Verwendung des monoklonalen Primärantikörpers durch ein entsprechendes Anti-Maus-IgG (1:3000, Sigma, Deisenhofen, Deutschland) mit Hilfe der Chemilumineszenz-Methode (SuperSignal Chemiluminescent Substrate; Pierce Chemical, Rockford, IL, USA) und nachfolgender Autoradiographie (Hyperfilm^{™} MP, Amersham Pharmacia Biotech, Buckinghamshire, England) nachgewiesen. Der Auftrag und Transfer gleicher Proteinmengen wurde nach "Stippen" der Transfermembran (5 Minuten 0.2 N NaOH, nachfolgend 3 x 10 Minuten Waschen mit H₂O durch Nachweis gleicher Proteinbanden von β-Actin mit einem monoklonalen primären Antikörper und einem Peroxidase-gekopplelten Anti-Maus IgG (beide von Sigma-Aldrich, 1:3000 Verdünnung) gezeigt.

### 7. Statistische Analyse

Wenn nicht anders angezeigt, sind alle Daten in Figuren und Text als Mittelwert ± SEM von n Experimenten angegeben. Die statistische Auswertung wurde mit dem Students t-Test für ungepaarte Daten mit einem P-Wert <0.05, der als statistisch signifikant angesehen wurde, durchgeführt.

### 8. Tierexperimenteller Nachweis der Decoy-Oligonukleotid-Wirkung

### 8.1 Maus

Zum Nachweis der Wirksamkeit des in der vorliegenden Anmeldung entwickelten Decoy-Oligonukleotid-basierten Therapieansatzes ist für die Indikation Antigen-induzierte Arthritis eine tierexperimentelle *Proof-of-concept-*Studie in der Maus mit 8-10 Tieren pro Gruppe durchgeführt worden (Modell siehe Henzgen et al., Exp. Toxicol. Pathol., (1996), 48, 255). Einmalige Applikation von 0,25 nmol des STAT-1-Decoy-Oligonukleotids (SEQ ID NO: 33) direkt in das Gelenk (intraartikuläre Injektion) reduzierte hochsignifikant über einen Zeitraum von 3-14 Tagen die Antigen-induzierte Gelenkschwellung (um 35%), die Intensität der Entzündungsreaktion (um 70%), die Gelenkdestruktion (um 80%), den Arthritis-Score insgesamt (um 70%) und die Konzentration pro-inflammatorischer Zytokine im Serum (z.B. Interleukin-6 um 80%). Das entsprechende Kontroll-Oligonukleotid hatte dagegen keinen therapeutischen Effekt.

Bemerkenswert an dieser Studie war zudem, dass die 14 Tage nach Induktion der Arthritis in der Haut ausgelöste Kontaktdermatitis (Typ-IV-Reaktion) - dabei wird das Antigen den Tieren noch einmal subkutan injiziert - bei den Decoy-Oligonukleotid-behandelten Mäusen ebenfalls hochsignifikant gehemmt war (um 35%).

### 8.2 Meerschweinchen

Nach zweimaliger Sensibilisierung der Meerschweinchen (Hartley, männlich, 350 g Körpergewicht) über einen Zeitraum von 7 Tagen (an Tag 1 in ein Ohr, an Tag 2 in das andere Ohr mit jeweils 50 µl einer 10%igen DNCB-Lösung in 50% Aceton/50% Olivenöl; an Tag 7 Boost in die Nackenhaut mit 15 µl einer 2%igen DNCB-Lösung in 95% Aceton/5% Olivenöl) wird die Kontakt-Dermatitis am Tag 13 durch die erneute Applikation von 2,4-Dinitrochlorobenzol (DNCB; 10 µl einer 0,5%igen Lösung von DNCB in 95% Aceton/5% Olivenöl) auf einem bzw. mehreren, circa 1 cm2 großen Arealen auf dem rasierten Rücken der Tiere ausgelöst und 24 Stunden später makroskopisch und histologisch beurteilt. Die so induzierte Kontakt-Dermatitis ist histologisch (Giemsa-Färbung) gekennzeichnet durch eine ausgeprägte Ödembildung und Spongiose im Bereich der Epidermis, eine Zunahme apoptotischer Zellen sowie eine massive Leukozyten-Infiltration (Figur 8). Intradermale Applikation eines STAT-1 Decoy-Oligonukleotids (SEQ ID NO: 19) nicht aber eines mutierten Kontroll-Oligonukleotids (5'-TGTGGACCGTAGGAAGTG-3',SEQ ID NO: 61) 1 Stunde vor der finalen DNCB-Exposition führte zu einer deutlichen Reduktion der genannten histologischen Parameter, d.h. insgesamt zu einer signifikanten Abschwächung der Entzündungsreaktion.

## Patentansprüche

1. Decoy-Oligonukleotid, wobei ein Nukleinsäurestrang eine Sequenz gemäß SEQ ID NO: 19 oder 20 aufweist.

2. Decoy-Oligonukleotid gemäß Anspruch 1, wobei das Decoy-Oligonukleotid modifizierte Intemukleotidbindungen aufweist.

3. Decoy-Oligonukleotid gemäß Anspruch 1 oder 2 als Arzneimittel.

4. Verwendung des Decoy-Oligonukleotid gemäß Anspruch 1 oder 2 als Arzneimittel zur Prävention und/oder Therapie kardiovaskulärer Komplikationen insbesondere Restenose nach perkutaner Angioplastie und Stenosierung von Venenbypässen, der Transplantatabstoßung, der *graft versus host disease* (GVHD), dem Ischämie/Reperfusionsschaden bei chirurgischen Operationen, von immunologischen Überempfindlichkeitsreaktionen (Typ I bis V), Autoimmunerkrankungen insbesondere Diabetes mellitus, multiple Sklerose und rheumatoide Arthritis, allen Formen akuter, subakuter und chronischer Entzündungserkrankungen insbesondere der Gelenke insbesondere Arthritis, der Atmungsorgane insbesondere Asthma bronchiale und chronische Bronchitis, der Haut insbesondere Psoriasis und Neurodermitis und des Gastrointestinaltrakts insbesondere Colitis ulcerosa und Morbus Crohn sowie dem septischen Schock.

## Claims

1. Decoy-oligonucleotide, wherein a nucleic acid strand exhibits a sequence according to SEQ ID NO: 9 or 20.

2. Decoy-oligonucleotide according to claim 1, wherein the decoy-oligonucleotide exhibits modified internucleotide bonds.

3. Decoy-oligonucleotide according to claim 1 or 2 as a medicament.

4. Use of the decoy-oligonucleotide according to claim 1 or 2 as a medicament for prevention and/or therapy of cardio-vascular complications, in particular ofrestenosis after percutaneous angioplasty and stenosis of venous bypasses, the transplant rejection, the graft versus host disease (GVHD), the ischemia/refusion related damage in context of surgical interventions, of immunological hypersensitivity reactions (type I to V), auto immune diseases, particularly diabetes mellitus, multiple sclerosis and rheumatoid arthritis, all forms of acute, sub-acute and chronic inflammatory diseases, in particular of the joints, particularly arthritis, of the respiratory organs, in particular bronchial asthma and chronic bronchitis, of the skin, in particular psoriasis and neurodermitis and of the gastrointestinal tract, particularly ulcerative colitis and Crohn's disease as well as of the septic shock.

## Revendications

1. Oligonucléotide leurre, dans lequel un brin d'acide nucléique présente une séquence selon SEQ ID NO: 19 ou 20.

2. Oligonucléotide leurre selon la revendication 1, dans lequel l'oligonucléotide leurre présente des liaisons internucléotidiques modifiées.

3. Oligonucléotide leurre selon la revendication 1 ou 2, en tant que médicament.

4. Utilisation de l'oligonucléotide leurre selon la revendication 1 ou 2, en tant que médicament destiné à la prévention et/ou la thérapie de complications cardiovasculaires, en particulier la resténose après angioplastie percutanée et formation de sténoses de pontages veineux, du rejet de transplant, de la maladie du greffon contre l'hôte (GVHD), de la lésion d'ischémie / reperfusion dans les interventions chirurgicales, des réactions d'hypersensibilité immunologiques (types I à V), des maladies auto-immunes, en particulier le diabète sucré, la sclérose en plaques et la polyarthrite rhumatoïde, de toutes les formes de maladies inflammatoires aiguës, subaiguës et chroniques en particulier des articulations, en particulier l'arthrite, des organes respiratoires, en particulier l'asthme bronchique et la bronchite chronique, de la peau, en particulier le psoriasis et la neurodermite, et du tractus gastro-intestinal en particulier la colite ulcéreuse et la maladie de Crohn, ainsi que le choc septique.


### SEQUENCE LISTING

<110> Avontec GmbH
<120> Modulation der Expression STAT-1-abhängiger Gene
<130> HEC-003 PCT
<140> unbekannt
   <141> 2002-10-02
<150> DE 101 48 828.9
   <151> 2001-10-04
<160> 61
<170> Patent In version 3.1
<210> 1
   <211> 11
   <212> DNA
   <213> Artificial
<220>
   <221> misc_feature
   <222> (1) .. (1)
   <223> g,a,c or t
<220>
   <221> misc_feature
   <222> (4) .. (4)
   <223> g,a,c or t
<220>
   <221> misc_feature
   <222> (11)..(11)
   <223> g,a,c or t
<400> 1
   nttncbgdaa n 11
<210> 2
   <211> 11
   <212> DNA
   <213> Artificial
<220>
   <221> misc_feature
   <222> (1)..(1)
   <223> g,a,c or t
<220>
   <221> misc_feature
   <222> (8)..(8)
   <223> g,a,c or t
<220>
   <221> misc_feature
   <222> (11) .. (11)
   <223> g,a,c or t
<400> 2
   ntthcvgnaa n 11
<210> 3
   <211> 11
   <212> DNA
   <213> Artificial
<400> 3
   attaccggaa g 11
<210> 4
   <211> 11
   <212> DNA
   <213> Artificial
<400> 4
   cttccggtaa t 11
<210> 5
   <211> 11
   <212> DNA
   <213> Artificial
<400> 5
   attccggtaa g 11
<210> 6
   <211> 11
   <212> DNA
   <213> Artificial
<400> 6
   cttaccggaa t 11
<210> 7
   <211> 11
   <212> DNA
   <213> Artificial
<400> 7
   attcctggaa g 11
<210> 8
   <211> 11
   <212> DNA
   <213> Artificial
<400> 8
   cttccaggaa t 11
<210> 9
   <211> 11
   <212> DNA
   <213> Artificial
<400> 9
   attcctgtaa g 11
<210> 10
   <211> 11
   <212> DNA
   <213> Artificial
<400> 10
   cttacaggaa t 11
<210> 11
   <211> 11
   <212> DNA
   <213> Artificial
<400> 11
   gttccaggaa c 11
<210> 12
   <211> 11
   <212> DNA
   <213> Artificial
<400> 12
   gttcctggaa c 11
<210> 13
   <211> 11
   <212> DNA
   <213> Artificial
<400> 13
   gttcccggaa g 11
<210> 14
   <211> 11
   <212> DNA
   <213> Artificial
<400> 14
   cttccgggaa c 11
<210> 15
   <211> 11
   <212> DNA
   <213> Artificial
<400> 15
   gttccgggaa c 11
<210> 16
   <211> 11
   <212> DNA
   <213> Artificial
<400> 16
   gttcccggaa c 11
<210> 17
   <211> 21
   <212> DNA
   <213> Artificial
<400> 17
   tgtgaattac cggaagtgag a 21
<210> 18
   <211> 21
   <212> DNA
   <213> Artificial
<400> 18
   tctcacttcc ggtaattcac a 21
<210> 19
   <211> 18
   <212> DNA
   <213> Artificial
<400> 19
   tgtgaattac cggaagtg 18
<210> 20
   <211> 18
   <212> DNA
   <213> Artificial
<400> 20
   cacttccggt aattcaca 18
<210> 21
   <211> 21
   <212> DNA
   <213> Artificial
<400> 21
   agtcagttcc aggaactgac t 21
<210> 22
   <211> 21
   <212> DNA
   <213> Artificial
<400> 22
   agtcagttcc tggaactgac t 21
<210> 23
   <211> 23
   <212> DNA
   <213> Artificial
<400> 23
   atgtgagttc ccggaagtga act 23
<210> 24
   <211> 23
   <212> DNA
   <213> Artificial
<400> 24
   agttcacttc cgggaactca cat 23
<210> 25
   <211> 18
   <212> DNA
   <213> Artificial
<400> 25
   acagttccgg gaactgtc 18
<210> 26
   <211> 18
   <212> DNA
   <213> Artificial
<400> 26
   gacagttccc ggaactga 18
<210> 27
   <211> 19
   <212> DNA
   <213> Artificial
<400> 27
   gacagttccg ggaactgtc 19
<210> 28
   <211> 19
   <212> DNA
   <213> Artificial
<400> 28
   gacagttccc ggaactgtc 19
<210> 29
   <211> 18
   <212> DNA
   <213> Artificial
<400> 29
   gtgtattccg gtaagtga 18
<210> 30
   <211> 18
   <212> DNA
   <213> Artificial
<400> 30
   tcacttaccg gaatacac 18
<210> 31
   <211> 21
   <212> DNA
   <213> Artificial
<400> 31
   ttatgtgaat tcctggaagt g 21
<210> 32
   <211> 21
   <212> DNA
   <213> Artificial
<400> 32
   cacttccagg aattcacata a 21
<210> 33
   <211> 25
   <212> DNA
   <213> Artificial
<400> 33
   catgttatgc atattcctgt aagtg 25
<210> 34
   <211> 25
   <212> DNA
   <213> Artificial
<400> 34
   cacttacagg aatatgcata acatg 25
<210> 35
   <211> 21
   <212> DNA
   <213> Artificial
<400> 35
   tgtgaattcc tgtaagtgag a 21
<210> 36
   <211> 21
   <212> DNA
   <213> Artificial
<400> 36
   tctcacttac aggaattcac a 21
<210> 37
   <211> 18
   <212> DNA
   <213> Artificial
<400> 37
   tgcatattcc tgtaagtg 18
<210> 38
   <211> 18
   <212> DNA
   <213> Artificial
<400> 38
   cacttacagg aatatgca 18
<210> 39
   <211> 15
   <212> DNA
   <213> Artificial
<400> 39
   atattcctgt aagtg 15
<210> 40
   <211> 15
   <212> DNA
   <213> Artificial
<400> 40
   cacttacagg aatat 15
<210> 41
   <211> 22
   <212> DNA/RNA
   <213> Artificial
<400> 41
   taccactgag acatcctgcc ac 22
<210> 42
   <211> 18
   <212> DNA/RNA
   <213> Artificial
<400> 42
   aacatcattg gcacgcag 18
<210> 43
   <211> 15
   <212> DNA/RNA
   <213> Artificial
<400> 43
   gtgaacctgc tccag 15
<210> 44
   <211> 24
   <212> DNA
   <213> Artificial
<400> 44
   cagagttcac tgaaacggaa tgcc 24
<210> 45
   <211> 20
   <212> DNA
   <213> Artificial
<400> 45
   tgcctgcctg ttgcacaacc 20
<210> 46
   <211> 20
   <212> DNA
   <213> Artificial
<400> 46
   agcaaggcat gatgttaacc 20
<210> 47
   <211> 20
   <212> DNA
   <213> Artificial
<400> 47
   gcattcctct cttccagagc 20
<210> 48
   <211> 19
   <212> DNA
   <213> Artificial
<400> 48
   tcttaatcag tggtggaag 19
<210> 49
   <211> 18
   <212> DNA
   <213> Artificial
<400> 49
   tttggtcaag ttgtttcc 18
<210> 50
   <211> 22
   <212> DNA
   <213> Artificial
<400> 50
   gtactccaca ttcctacttc tc 22
<210> 51
   <211> 22
   <212> DNA
   <213> Artificial
<400> 51
   tttgggtcta ttccgttgtg tc 22
<210> 52
   <211> 20
   <212> DNA
   <213> Artificial
<400> 52
   gttcatccgg caccagtcag 20
<210> 53
   <211> 21
   <212> DNA
   <213> Artificial
<400> 53
   acgtgcacat gagctgccta c 21
<210> 54
   <211> 28
   <212> DNA
   <213> Artificial
<400> 54
   gcggatcccc tccagcatga aagtctct 28
<210> 55
   <211> 28
   <212> DNA
   <213> Artificial
<400> 55
   acgaattctt cttgggttgt ggagtgag 28
<210> 56
   <211> 23
   <212> DNA
   <213> Artificial
<400> 56
   gtgcatttcc cgtaaatctt gtc 23
<210> 57
   <211> 27
   <212> DNA
   <213> Artificial
<400> 57
   cacttgataa cagaaagtga taactct 27
<210> 58
   <211> 22
   <212> DNA
   <213> Artificial
<400> 58
   agttgagggg actttcccag gc 22
<210> 59
   <211> 19
   <212> DNA
   <213> Artificial
<400> 59
   gacagtgcag tgaactgtc 19
<210> 60
   <211> 25
   <212> DNA
   <213> Artificial
<400> 60
   catgttatgc agaccgtagt aagtg 25
<210> 61
   <211> 18
   <212> DNA
   <213> Artificial
<400> 61
   tgtggaccgt aggaagtg 18